(19) **Europäisches Patentamt / European Patent Office / Office européen des brevets**

(11) **EP 1 740 699 B1**

(12) **EUROPEAN PATENT SPECIFICATION**

(45) Date of publication and mention
of the grant of the patent:
**02.03.2011 Bulletin 2011/09**

(21) Application number: **05739064.3**

(22) Date of filing: **28.04.2005**

(51) Int Cl.:
*C12N 15/12* (2006.01)   *G01N 33/68* (2006.01)
*C12Q 1/68* (2006.01)   *A61K 48/00* (2006.01)
*A61P 9/06* (2006.01)

(86) International application number:
**PCT/CA2005/000646**

(87) International publication number:
**WO 2005/105997 (10.11.2005 Gazette 2005/45)**

(54) **CONNEXIN 40 TISSUE SPECIFIC GENE MUTATIONS**

GEWEBESPEZIFISCHE CONNEXIN-40-GENMUTATIONEN

MUTATIONS GENETIQUES SPECIFIQUES A UN TISSU DE CONNEXINE 40

(84) Designated Contracting States:
**AT BE BG CH CY CZ DE DK EE ES FI FR GB GR HU IE IS IT LI LT LU MC NL PL PT RO SE SI SK TR**

(30) Priority: **29.04.2004 US 566355 P**

(43) Date of publication of application:
**10.01.2007 Bulletin 2007/02**

(73) Proprietors:
- **GOLLOB, MIchael H.**
  **Ottawa, ON K1Y 1J1 (CA)**
- **Jones, Douglas L.**
  **London, ON N5X 4N6 (CA)**
- **Krahn, Andrew D.**
  **London, ON N6H 5B4 (CA)**

(72) Inventors:
- **GOLLOB, MIchael H.**
  **Ottawa, ON K1Y 1J1 (CA)**
- **Jones, Douglas L.**
  **London, ON N5X 4N6 (CA)**
- **Krahn, Andrew D.**
  **London, ON N6H 5B4 (CA)**

(74) Representative: **Holmberg, Martin Tor**
**Bergenstråhle & Lindvall AB**
**P.O. Box 17704**
**118 93 Stockholm (SE)**

(56) References cited:
**CA-A1- 2 260 756**

- **GROENEWEGEN W A ET AL: "A CARDIAC SODIUM CHANNEL MUTATION COSEGREGATES WITH A RARE CONNEXIN40 GENOTYPE IN FAMILIAL ATRIAL STANDSTILL" CIRCULATION RESEARCH, GRUNE AND STRATTON, BALTIMORE, US, vol. 92, 1 January 2003 (2003-01-01), pages 14-22, XP008075881 ISSN: 0009-7330**
- **KIRCHHOFF S ET AL: "REDUCED CARDIAC CONDUCTION VELOCITY AND PREDISPOSITION TO ARRHYTHMIAS IN CONNEXIN40-DEFICIENT MICE" CURRENT BIOLOGY, CURRENT SCIENCE, GB, vol. 8, no. 5, 26 February 1998 (1998-02-26), pages 299-302, XP001182395 ISSN: 0960-9822**
- **KANTER H L ET AL: "MOLECULAR CLONING OF TWO HUMAN CARCIAC GAP JUNCTION PROTEINS, CONNEXIN40 AND CONNEXIN45" JOURNAL OF MOLECULAR AND CELLULAR CARDIOLOGY, XX, XX, vol. 26, no. 7, 1 January 1994 (1994-01-01), pages 861-868, XP000905086 ISSN: 0022-2828**
- **VAN DER VELDEN HUUB MW ET AL: "CARDIAC GAP JUNCTIONS AND CONNEXINS: THEIR ROLE IN ATRIAL FIBRILLATION AND POTENTIAL AS THERAPEUTIC TARGETS" CARDIOVASCULAR RESEARCH, XX, XX, vol. 54, 1 January 2002 (2002-01-01), pages 270-279, XP008075880 ISSN: 0008-6363**

EP 1 740 699 B1

- MUSA HASSAN ET AL: "AMINO TERMINAL GLUTAMATE RESIDUES CONFER SPERMINE SENSITIVITY AND AFFECT VOLTAGE GATING AND CHANNEL CONDUCTANCE OF RAT CONNEXIN40 GAP JUNCTIONS" JOURNAL OF PHYSIOLOGY, XX, XX, vol. 557, no. 3, 23 April 2004 (2004-04-23), pages 863-878, XP008075925 ISSN: 0022-3751
- KRUTOVSKIKH V ET AL: "Connexin gene mutations in human genetic diseases." MUTATION RESEARCH, vol. 462, no. 2-3, April 2000 (2000-04), pages 197-207, XP002493313 ISSN: 0027-5107
- GROENEWEGEN W.A. ET AL: 'A cardiac sodium channel mutation cosegregates with a rare connexin40 genotype in familial atrial standstill.' CIRCULATION RESEARCH. vol. 92, 2003, pages 14 - 22, XP008075881
- KIRCHHOFF S. ET AL: 'Reduced cardiac conduction velocity and predisposition to arrhythmias in connexin40-deficient mice.' CURRENT BIOLOGY. vol. 8, 1998, pages 299 - 302, XP001182395
- VAN DER VELDEN H.M.W. ET AL: 'Cardiac gap junctions and connexins: their role in atrial fibrillation and potential as therapeutic targets.' CARDIOVASCULAR RESEARCH. vol. 54, 2002, pages 270 - 279, XP008075880
- KANTER H.L. ET AL: 'Molecular cloning of two human cardiac gap junction proteins, connexin40 and connexin45.' J MOL CELL CARDIOL. vol. 26, 1994, pages 861 - 868, XP000905086

**Description**

**FIELD OF INVENTION**

The present invention relates to detecting and treating cardiac arrhythmia in a subject.

**BACKGROUND OF THE INVENTION**

Atrial fibrillation (AF) is characterized by rapid, erratic electrical activation of atrial myocardium. Consequently, effective atrial contractility is lost, promoting clot formation and a significant risk of stroke. The rapid activity of the atria may be conducted to the ventricles, resulting in deterioration of heart function. In addition to causing substantial morbidity, AF confers an increased mortality risk, independent of co-existing risk factors. In the United States, over 2 million adults are affected with AF, the prevalence increasing with age (5.9% at age > 65 years). Thus, the socioeconomic burden of disease management is considerable.

Recent studies on the molecular etiology for AF have focused on familial forms of the disease. For example, a mutation in KCNQ1, a potassium channel also implicated in a form of Long QT Syndrome, has been identified as the molecular basis of autosomal dominant AF in a single family from Shandong Province, China (Chen, Y.H. et. al., Science 299, 251-254; 2003). Two families demonstrating autosomal dominant AF have been mapped to 10q22-q24 and 6p14-p16, respectively (Brugada, R. et. al., N. Engl. J. Med. 336, 905-911,1997; Ellinor, P.T., et. al., Circulation 107, 2880-2883; 2003). However, a causal gene has not been identified.

Connexin40 (Cx40) is a gap junction protein with restricted atrial and conduction system expression in the human heart. connexin40 is known to play a critical role in mediating coordinated electrical activation of myocardium through intercellular coupling (Kanno, S. & Saffitz, J.E. Cardiovasc. Pathol. 10, 169-177, 2001). Huub, M.W. et. al., (2002, Cardiovascular Research 54; 270-279) suggest that gap junctional remodelling including a decrease in the connexin40:Cx43 protein ratio may be involved in the stabilization of atrial fibrillation. However, there is no disclosure of specific mutations in Connexin40 in this publication.

Alcolea, S et. al., (2004, Circ Res. 94:100-109) discloses the generation of connexin40 knock out mice that are viable and fertile. The knock out mice exhibit increased incidence of inducible atrial tachyarrhythmias.

Ri et al., (1999, Biophysical Journal 76: 2887-2898) discloses amino acid substitutions at position 87 and 86 of Connexin 32 (Cx32). However, there is no disclosure of any mutations in the nucleotide sequence of connexin40.

Suchyna et al., (1993, Nature 365:847-849) disclose that a mutation of proline 87 in Connexin 26 (Cx26) causes a reversal in the voltage gating response when the mutant hemichannel is paired with wild type Cx26 in the *Xenopus* oocyte system. Specific mutations disclosed in this reference are Cx26P87L, Cx26P87I, Cx26P87A, Cx26P87G and Cx26P87V. There is no teaching of mutations in the nucleotide sequence of connexin40.

WO 02/19966 (Donahue J.K. and Marban E.) discloses methods of preventing or treating cardiac arrhythmia, including administering one or more polynucleotides that modulate an electrical property of the heart. There is no teaching of any mutations in the connexin40 gene sequence.

**SUMMARY OF THE INVENTION**

The present invention relates to detecting and identifying a compound for the treatment of cardiac arrhythmia in a subject.

It is an object of the invention to provide an improved method for identifying a compound for the treatment ofcardiac arrhythmia.

According to the present invention there is provided a method (A) of detecting cardiac arrhythmia, or the potential for cardiac arrhythmia, in a subject comprising, determining whether there is a mutation in the nucleotide sequence, the amino acid sequence, or both, of connexin40 obtained from the subject, wherein the mutation in the nucleotide sequence, the amino acid sequence, or both comprises a Pro88Ser mutation, an Ala96Ser mutation, or a Gly38Asp mutation in connexin40 (SEQ ID NO:1). For example, the presence of the mutation may be determined by a method selected from the group consisting of

comparing the nucleotide sequence of connexin40 obtained from the subject to a nucleotide sequence of connexin40 obtained from a healthy patient using a sequence comparison,

comparing the nucleotide sequence of connexin40 obtained from the subject to a nucleotide sequence of connexin40 obtained from a healthy patient, or otherwise determining if there is a mutation in connexin40, using PCR,

comparing the nucleotide sequence of connexin40 obtained from the subject to a nucleotide sequence of connexin40 obtained from a healthy patient, or otherwise determining if there is a mutation in connexin40, using LCR,

comparing the nucleotide sequence of connexin40 obtained from the subject to a nucleotide sequence of connexin40 obtained from a healthy patient, or otherwise determining if there is a mutation in connexin40, using SNP analysis,

comparing the nucleotide sequence of connexin40 obtained from the subject to a nucleotide sequence of connexin40 obtained from a healthy patient, or otherwise determining if there is a mutation in connexin40, using a nucleic acid array,

comparing the amino acid sequence of connexin40 obtained from the subject to an amino acid sequence of connexin40 obtained from a healthy patient, or otherwise determining if there is a mutation in connexin40, using an epitope-based assay,

comparing the amino acid sequence of connexin40 obtained from the subject to an amino acid sequence of connexin40 obtained from a healthy patient, or otherwise determining if there is a mutation in connexin40, using a monoclonal antibody,

comparing the amino acid sequence of connexin40 obtained from the subject to an amino acid sequence of connexin40 obtained from a healthy patient, or otherwise determining if there is a mutation in connexin40, using an ELISA assay, and

comparing the amino acid sequence of connexin40 obtained from the subject to an amino acid sequence of connexin40 obtained from a healthy patient, or otherwise determining if there is a mutation in connexin40, using western analysis.

The present invention is also directed to the method (A) as defined above wherein the mutation in the nucleotide sequence encodes a mutation within a transmembrane domain. Alternatively, the mutation in the nucleotide sequence may encode a mutation between amino acids 20-42, amino acids 76-98, amino acids 160-183, amino , or a combination thereof, of connexin40. Furthermore, the mutation in the nucleotide sequence encodes a mutation selected from the group consisting of a Pro88Ser mutation, an Ala96Ser mutation, a Gly38Asp mutation,. Preferably, the nucleotide sequence of connexin40 is determined from a blood sample, or a heart tissue sample, obtained from the patient.

Also described herein is an isolated nucleic acid comprising the sequence selected from the group consisting of wt connexin40, a modified Cx40 comprising a C to T mutation at position 262, a modified Cx40 comprising an A to G mutation at position 487, a modified Cx40 comprising an G to A mutation at position 113, and a modified Cx40 comprising an G to T mutation at position 286.

Also described herein is a nucleic acid array comprising one or more than one of a nucleic acid comprising the sequence selected from the group consisting of wt connexin40, a modified connexin40 comprising a C to T mutation at position 262, a modified connexin40 comprising an A to G mutation at position 487, a modified connexin40 comprising an G to A mutation at position 113, and a modified connexin40 comprising an G to T mutation at position 286.

Furthermore, also described herein is a method (B) of detecting cardiac arrhythmia, or a potential for cardiac arrhythmia in a subject comprising, obtaining a nucleic acid sample from the subject and hybridizing the nucleic acid sample with the nucleic acid array as described above. Preferably, the nucleic acid sample is obtained from blood or heart tissue of the subject.

In addition also described herein is a method (C) of restoring intracellular trafficking in a cell that is deficient in gap junctions comprising, introducing a wild type connexin40 gene into the cell, and expressing the wild type connexin40 gene. The wild type connexin40 gene may be introduced in the cell using a viral vector.

Furthermore, also described is a method (D) of detecting cardiac arrhythmia comprising determining whether there is impaired intracellular trafficking, impaired electrical coupling, or both intracellular trafficking and impaired electrical coupling, between cells obtained from a heart tissue.

Furthermore, also described herein is a method (E) of identifying a compound for the treatment of cardiac arrhythmia, or the potential for cardiac arrhythmia, comprising,

- providing a cell culture expressing a modified connexin40, the modified connexin40 comprising a Pro88Ser mutation, as Ala96Ser mutation or a Gly38Asp mutation in SEQ ID NO:1, the cell culture exhibiting impaired intracellular trafficking, impaired electrical coupling, reduced gap junction plaque formation, reduced intracellular coupling, or a combination thereof, when compared to a cell expressing wild-type connexin40 (SEQ ID NO:1);

- adding the compound to the cell culture; and

- determining if intracellular trafficking is improved, electrical coupling is improved, gap junction plaques are formed, intracellular coupling is improved, electrical coupling, or a combination thereof, when compared to an untreated cell culture expressing the modified connexin40.

In the present invention, "idiopathic" AF has been identified as having a genetic basis, and mutations that predispose the atria to fibrillation have been found to be somatic in nature.

This summary of the invention does not necessarily describe all features of the invention.

## BRIEF DESCRIPTION OF THE DRAWINGS

These and other features will become more apparent from the following description in which reference is made to the appended drawings wherein:

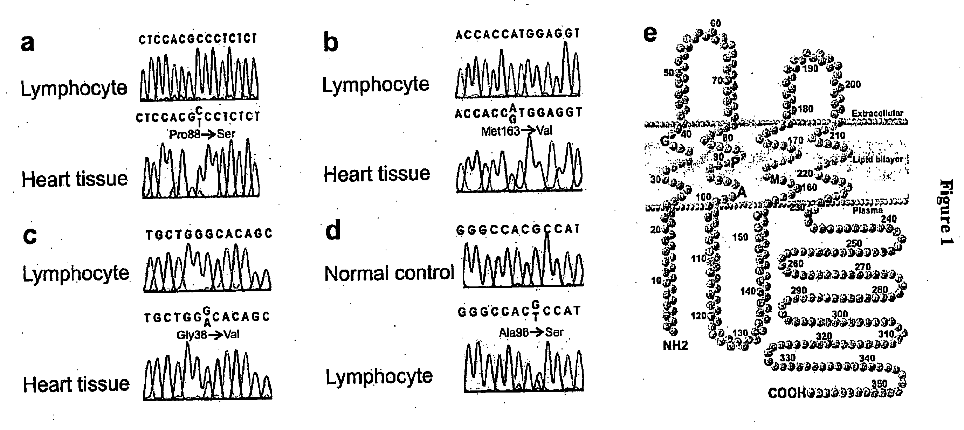

**Figure 1** Shows detected mutations of connexin40 in idiopathic atrial fibrillation. **Figure 1a**, shows sequence analyses of lymphocyte and heart specimen DNA from an affected individual harbouring a relatively lower abundance of mutant allele (C→T) compared to wild type allele, detected in the heart specimen. Allelic subcloning of PCR products confirmed the presence of mutant allele in 17% (4/24) of clones analyzed. The C→T mutation results in a proline for serine substitution at amino acid position 88. **Figure 1b**, shows sequence analyses from affected patient 9, demonstrating a 487A→G (Met163Val) mutation detected from heart specimen analysis, but not from the individuals lymphocyte DNA. Figure 1c, shows sequence analyses from the same affected patient 9 (see Figure 1b) demonstrating a 113G→A (Gly38Val) mutation detected from heart specimen analysis, but not from the individuals lymphocyte DNA. **Figure 1d**, shows sequence analysis showing the 286G→T (Ala96Ser) mutation detected from lymphocyte DNA in affected patient 6. The mutation was identified from both lymphocyte and heart specimens. **Figure 1e,** shows the location of mutated residues (G, P, A) in the predicted protein of connexin40. Gly38 is located within the first transmembrane domain (TM1), P88 and Ala96 within TM2, and Met163 within TM3.

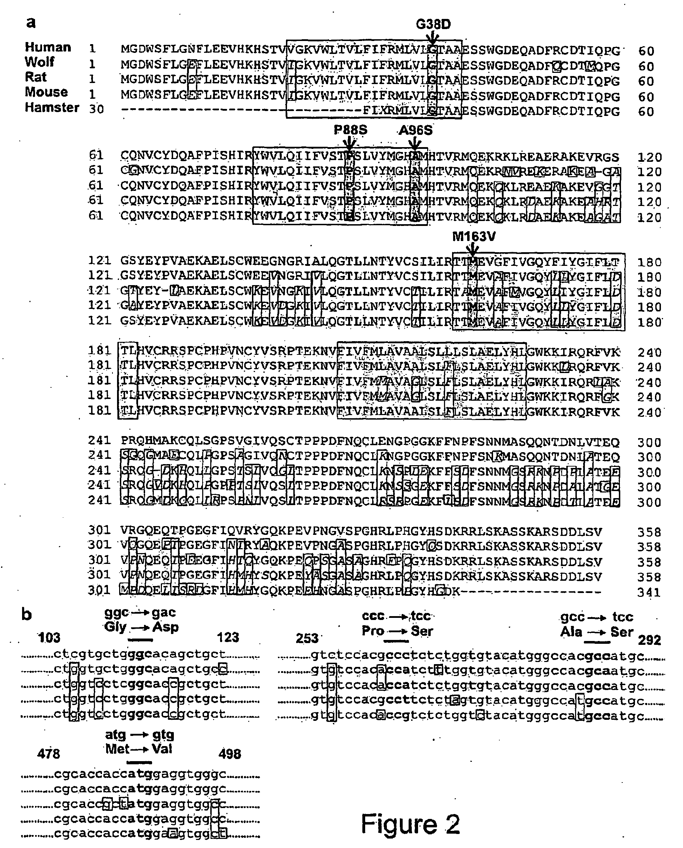

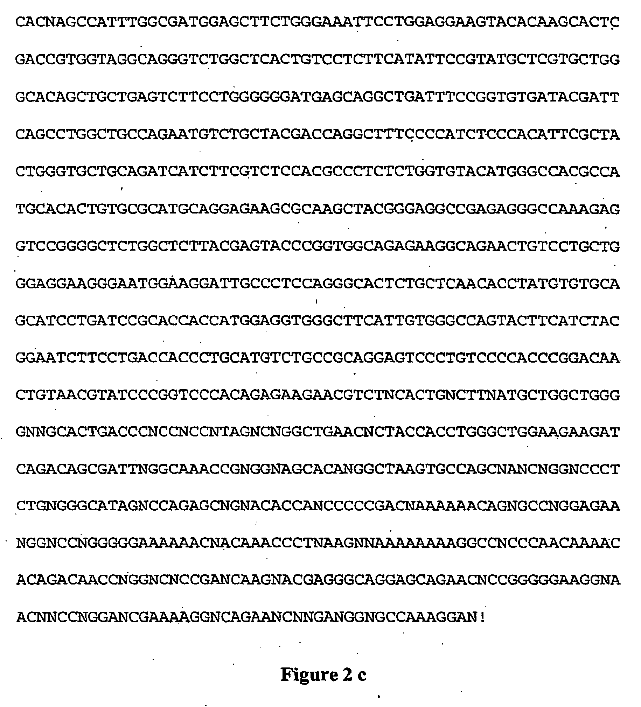

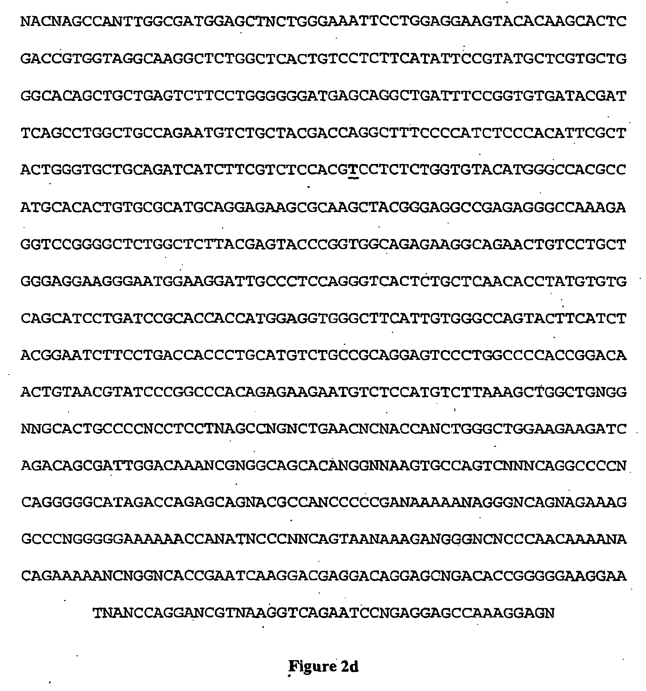

**Figure 2** shows amino acid sequence alignment of several mammalian connexin40. **Figure 2a**, shows the amino acid sequences of connexin40 for human (SEQ ID NO:1), wolf (SEQ ID NO:2), rat (SEQ ID NO:3), mouse (SEQ ID NO:4), and hamster (SEQ ID NO:5). Identified mutations in Connexin40 proteins are denoted in bold at positions 38 (G38V), 88 (P88S), 96 (A96S) and 163 (M163V). The identified mutations as described herein occur at conserved residues across species in which the structure is known. Non-conserved residues are indicated by boxed squares and occur predominantly in the C-terminal portion of the protein. Lightly shaded, large boxes indicate transmembrane spanning regions. **Figure 2b**, shows nucleotide alignment in the region of identified mutations. Sequence homology is demonstrated at the DNA level, including conservation of the specific nucleotides where mutations were identified in affected patients. **Figure 2c**, shows the nucleotide sequnce of wild-type connexin40 (SEQ ID NO:6). **Figure 2d**, shows the nucleotide sequence of a mutant connexin40 (SEQ ID NO:7) comprising a C to T (underlined) substitution at position 262, encoding the Pro88Ser mutation.

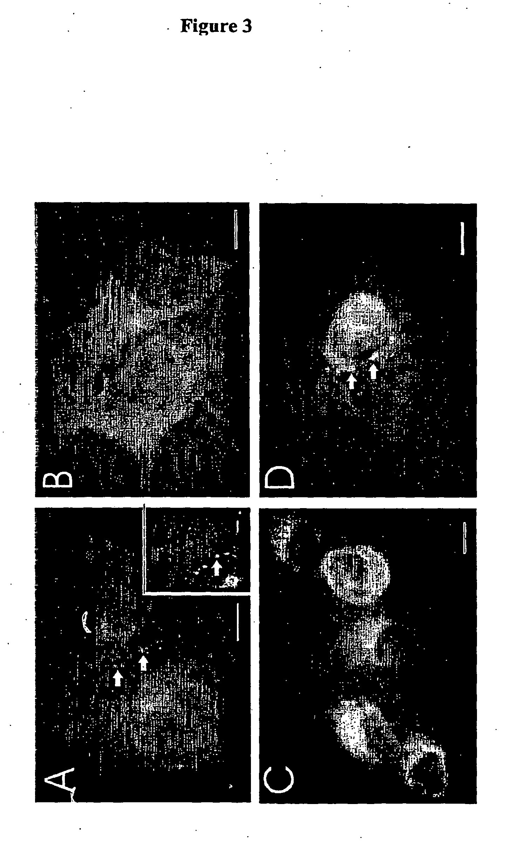

**Figure 3,** shows that a Pro88Ser connexin40 mutant is retained intracellularly when expressed in N2A cells (a gap junctional communication-deficient neuroblastoma cell line) which are normally intercellular communication-deficient. **Figure 3a**, shows wild-type connexin40 and GFP-tagged wild-type connexin40 (insert) assembled into gap junctions (arrows) at cell-cell interfaces. **Figure 3b,** shows that untagged Pro88Ser connexin40 mutants are retained in the intracellular compartment. **Figure 3c,** shows that GFP-tagged Pro88Ser connexin40 mutant are retained in the intracellular compartment. Figure 3d, shows that the Pro88Ser mutant was rescued and assembled into gap junctions at cell-cell interfaces in cells co-expressing wild-type connexin40 (arrows). Bar =10 μm

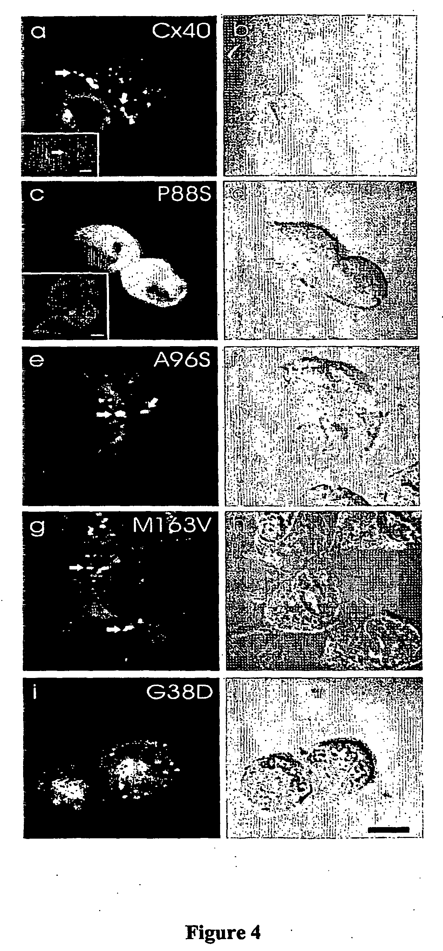

**Figure 4** shows differential localization of connexin40 mutants in N2A cells. N2A cells were transfected with cDNAs encoding GFP-tagged wild-type connexin40 **(Figures 4a, 4b),** or the GFP-tagged connexin40 mutants, Prol88Ser (P88S; **Figures 4c,4d),** Ala96Ser (A96S; **Figure 4e, 4f),** Met163Val (M163V; Figures 4g, 4h) or Gly38Asp (G38D; **Figures 4i, 4j).** GFP-tagged **(Figure 4c)** and untagged **(Figure 4c, insert)** P88S failed to assemble into gap junctions. The G38D mutant **(Figure 4i)** was primarily retained within intracellular compartments with occasional formation of a gap junction-like structure. Gap junction-like structures (arrows) were seen at cell-cell interfaces in cells expressing untagged connexin40 **(Figure 4a, insert),** GFP-tagged connexin40 (Figure 4a), A96S (Figure 4e) or M163V (Figure 4g). Transmitted light images reveal that all cells analyzed had contacting neighboring cells **(Figures 4b, 4d, 4f, 4h, 4j).** Bar = 10 um.

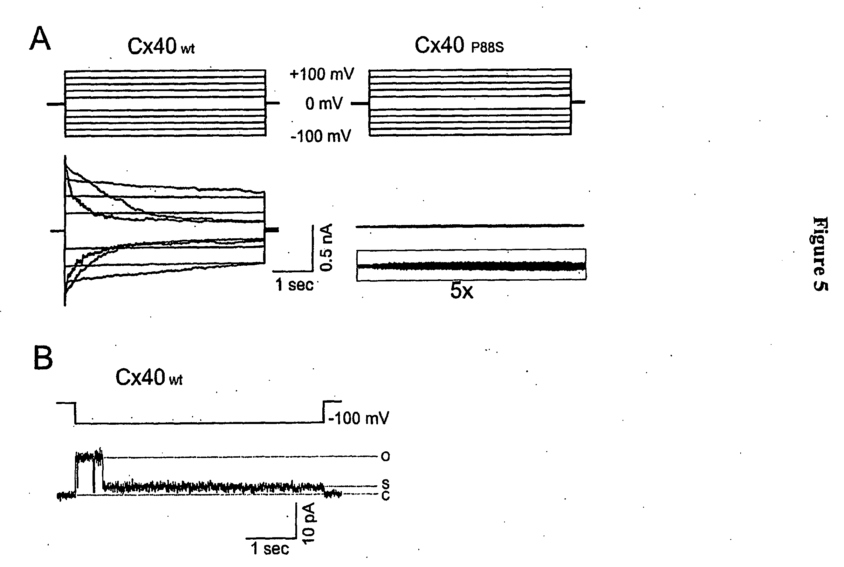

**Figure 5** shows that a Pro88Ser mutant and a Ala96Ser mutant in human connexin40 leads to impaired functional coupling between pairs of transfected N2A cells, and exert a dominant negative effect on wtconnexin40 activity. **Figure 5a**, shows the voltage protocol (-100 to +100 mV) used to test for coupling and voltage-dependent gating of connexin40 transfected N2A cell pairs (Top traces). Bottom traces are superimposed current records from one cell of the pair in response to the voltage protocol applied to the other cell. Coupling and voltage-dependent gating were observed in wtconnexin40 (left), but not in the Pro88Ser mutant connexin40 transfected N2A cells (right). Bottom right inset shows 5-times enlarged current records obtained from a transfected Pro88Ser connexin40 N2A cell pair. **Figure**

**5b,** shows that in weakly coupled N2A cell pairs transfected with wtconnexin40, single unitary current can be identified. Open state (O, single channel conductance $\gamma_j$ = 102 pS), subconductance state (S, $\gamma_{js}$ = 24 pS) and closed state (C) are illustrated. **Figure 5c** shows antisense-treated (AS) oocytes, injected with wtconnexin40, mutant connexin40 (Pro88Ser and Ala96Ser mutants), or co-injected with equal amounts of wt and mutant cRNA. The amount of wtconnexin40 injected into each oocyte was held constant to facilitate comparison of the homotypic and heteromeric oocyte pair data. Both Pro88Ser and Ala96Ser mutants demonstrated absent or minimal functional cell-cell coupling similar to results in N2A cells, and significantly impaired the activity of wtconnexin40. Data is presented as the mean ±SEM of the number of pairs indicated in parentheses for each condition.

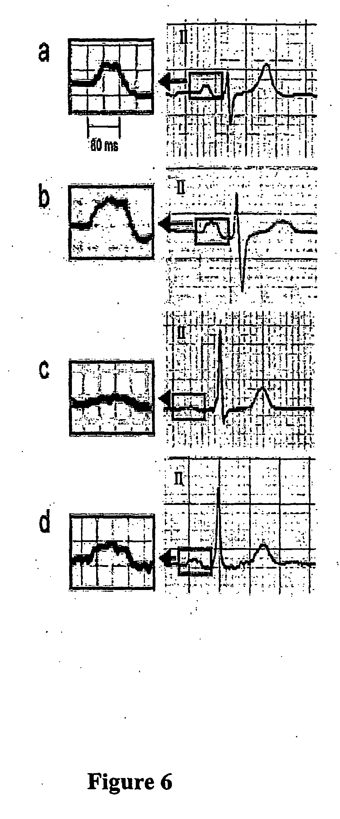

**Figure 6** shows surface electrocardiographic analysis of P-wave duration in family members of patient #6 harboring the Ala96Ser mutation. **Figure 6a** shows P-wave duration in the unaffected spouse, age 74, appears of normal morphology and duration. **Figure 6b** shows P-wave duration from an archived electrocardiogram of affected patient #6 during normal sinus rhythm appears broad with an abnormal P-wave duration in the range of 120 ms (Normal < 110 ms). **Figures 6c and 6d)** shows abnormal P-wave morphology and duration, indicative of prolonged atrial myocardial conduction time in the sons carrying the Ala96Ser mutation. Legend: Each small square represents 40 ms in duration.

## DETAILED DESCRIPTION

The present invention relates to detecting and treating cardiac arrhythmia in a subject.

The following description is of a preferred embodiment.

The present invention provides a method of detecting cardiac arrhythmia, or the potential for cardiac arrhythmia, in a subject comprising, determining whether there is one or more than one mutation in the nucleotide sequence of connexin40 (connexin40; also referred to GJA5). The presence of the one or more than one mutation may be determined using any techniques known in the art, for example but not limited to, by comparing the nucleotide sequence of connexin40 obtained from the subject, to a nucleotide sequence of connexin40 obtained from a healthy patient, using chip arrays comprising one or more than one mutation in connexin40, PCR, LCR or SNP assays known to detect single nucleic acid polymorphisms, or other techniques known in the art for detecting modification in a nucleotide sequence. Additionally, methods to detect changes in amino acids sequences may also be used including comparison of amino acids sequences, epitope based assays, antibody screening assays, ELISA, or western analysis. Preferably, the mutation in the nucleotide sequence of connexin40 modifies intracellular trafficking, electric coupling, or modifies intracellular trafficking and electric coupling. More preferably, the one or more than one mutation within connexin40 is within one or more than one transmembrane domain region, including between amino acids 20-42, 76-98, 160-183 and 206-230 of connexin40 (e.g. see Figures 1e, and 2a).

Furthermore, the present invention provides a method of detecting cardiac arrhythmia, or the potential for cardiac arrhythmia comprising, determining whether there is impaired intracellular trafficking, impaired electrical coupling, or modified intracellular trafficking and electrical coupling, between cells obtained from heart tissue. Methods as described herein, or any method as would be known to one of skill in the art may be used for such a determination including, but not limited to, detecting electrical coupling between the cells.

In order to determine the role that connexin40 may play in cardiac arrhythmia, genomic DNA was isolated from resected cardiac tissue from idiopathic atrial fibrillation (AF) patients. Sequencing of the gene encoding connexin40 identified heterozygous mutations in cardiac tissue (see Example 1). Mutations were identified within the transmembrane domains of connexin40. For example, a mutation in connexin40 (SEQ ID NO:6; Figure 2c) at position 262 of C to T (see SEQ ID NO:7; Figure 2d), predicting a Pro88Ser substitution, was identified in 4 patients (see Figure 1a). An additional patient harbored 2 non-allelic mutations leading to Gly38Asp (nucleotide 113 G to A; Figure 1b) and Met163Val (nucleotide 487A to G; Figure 1c) substitutions. Sequencing of DNA from peripheral lymphocytes of these patients showed no evidence of mutation, indicating a somatic source of the genetic defects. Cardiac specimens from an additional patient demonstrated a mutation at nucleotide 286 from G to T (Figure 1d), predicting an Ala96Ser amino acid change. This mutation was also identified from lymphocyte DNA of this affected patient.

The Pro88 residue is located in the second transmembrane domain of connexin40, and this residue is evolutionarily conserved in mammalian connexins (see Figure 2a). Cross-species comparison of connexin40 shows a > 80% homology of protein and protein-encoding DNA sequence, reflecting the evolutionary conservation of the connexin40 protein and gene structure (Figure 2a). An analogous mutation has been implicated in the transduction of voltage-gating in the connexin protein family, including Cx32 and Cx50 (Suchyna, T.M., et. al., Nature 365, 847, 1993; Nelis E. et. al., Hum. Mutat. 9, 47, 1997; Janssen E.A., et. al., Hum. Genet. 99, 501, 1997; Bort, S., et. al., Hum. Genet. 99, 746, 1997; Shiels A., et. al., Am. J. Hum. Genet. 62, 526, 1998; Kuntzer T., et. al., J. Neurol. Sci. 207, 77, 2003).

Mutations of Met 163 in Cx26 have been associated with hereditary deafness (Marlin, S. et. aL, Arch. Otolaryngol. Head Neck Surg. 127. 927, 2001; Bayazit, Y.A. et. al., Int. J. Pediatr. Otorhinolaryngol. 67, 1331, 2003). The Gly38Asp mutation has not been reported in other connexins known to cause human disease, but mutations in this region have been observed (Krutovskikh, V. & Yamasaki, H., Mutat. Res. 462, 197,2000).

Direct sequencing of 100 alleles from heart tissue in individuals unaffected by atrial fibrillation and 240 alleles from lymphocyte DNA of healthy controls showed no evidence of the Pro88Ser, Met163Val, or Gly38Asp mutations, further supporting their potential pathogenetic importance. However, the Ala96Ser mutation was identified in one healthy, 48 year old individual (allele frequency 0.5%). Without wishing to be bound by theory, this may indicate that this allele represents a rare, benign polymorphism or that this individual may be predisposed to developing AF in the future. This latter possibility is supported by the known prevalence of AF in patients 50-55 years of age (0.5%; Feinberg, W.M., et. al., Arch. Intern Med. 155, 469-473, 1995), and the observation that mutations at the identical position of Ala96 have been observed in deafness and CMT syndrome (Kelley, P.M., et. al., Am. J. Hum. Genet. 62, 792-799,1998; Bone, L.J. et. al., Neurology 45, 1863-1866 (1995).

Two further sequence variations from controls were identified, one at position 369 of SEQ ID NO:6, comprising a C to T and leading to a Tyr123 silent mutation, and the second at position 377, also comprising C to T, and causing a Pro 126Leu substitution. Pro126 is deleted in the rat connexin40 homologue (Figure 2) and occurs within the intracytoplasmic loop of connexin40, a region of known variability between connexin proteins (Kanno, S. & Saffitz, J.E., Cardiovasc. Pathol. 10, 169, 2001).

Therefore, described herein is a method of detecting cardiac arrhythmia, or the potential for cardiac arrhythmia, in a subject comprising, determining whether there is a mutation in the nucleotide sequence of connexin40 (connexin40) obtained from the subject. Preferably, the mutation in the nucleotide sequence of connexin40 modifies intracellular trafficking, electrochemical coupling, or both modifies intracellular trafficking and electrochemical coupling, for example but not limited to one or more than one mutation, within one or more than one transmembrane domain region, including between amino acids 20-42, 76-98, 160-183 and 206-230 of connexin40 (e.g. see Figures 1e, and 2a). Non-limiting examples of mutations within transmembrane domain regions include, but are not limited to substitution of amino acid:

- glycine to aspartate at position 38 of connexin40 (Gly38Asp; see Figure 1c);

- proline to serine at position 88 of connexin40(Pro88Ser; see Figure 1a);

- alanine to serine at position 96 of connexin40 (Ala96Ser; see Figure 1d), or

- methionine to valine at position 163 of connexin40 (Met163Val; see figure 1b).

Any method may be used to determine the nucleotide or amino acid sequence of the connexin40 gene or protein. Non-limiting examples include, but are not limited to comparing the nucleotide sequence of connexin40 obtained from the subject, to a nucleotide sequence of connexin40 obtained from a healthy patient, using nucleic acid arrays (e.g. DNA chip) comprising one or more than one nucleotide sequence encoding one or more than one mutation in connexin40 and probing the chip with either RNA or DNA obtained from the subject, PCR, LCR or SNP assays known to detect single nucleic acid changes obtained from the subject, or other techniques as would be known in the art for detecting modification in a nucleotide sequence. Alterations within the amino acid sequence of connexin40 may also be used to determine one or more than one mutation within connexin40. Methods to identify such changes include, but are not limited to epitope-based screens, monoclonal antibodies, antibody based assays, ELISA, or western analysis, as would be known to one of skill in the art (e.g. Sambrook et al., Molecular Cloning, A Laboratory Manual 2nd ed. 1989; Ausubel et al., eds., Current Protocols in Molecular Biology, 1994, which are incorporated herein by reference),.

Samples obtained from the subject for use in the above method, may be may be obtained from heart tissue, or blood, using methods as known in the art. For example ventricular sampling methods may be employed to obtain a sample of heart tissue, or the sample may be obtained from blood that is obtained within the heart, for example an atrial blood sample. However, it is also considered within the scope of the present invention that a blood sample may be obtained from other regions of the body.

Described herein is also a method of detecting cardiac arrhythmia or the potential for cardiac arrhythmia in a subject comprising, determining whether there is a mutation in the nucleotide sequence of connexin40 (connexin40) obtained from the subject using a nucleic acid array comprising one or more than one modified connexin40 nucleic acid. Preferably, the one or more than one nucleic acid comprises a mutation in the nucleotide sequence of connexin40 that encodes a protein resulting in a modification in intracellular trafficking, electrical coupling, or both. For example, which is not to be considered limiting, the one or more than one nucleic acid encodes a modified connexin40 protein comprising a mutation within one or more than one transmembrane domain region, including between amino acids 20-42, 76-98, 160-183 and 206-230 of connexin40 (Figures 1e, and 2a). Non-limiting examples of mutations within transmembrane domain regions include, but are not limited to, substitution of amino acid:

- glycine to aspartate at position 38 of connexin40 (Gly38Asp; see Figure 1c);

- proline to serine at position 88 of connexin40(Pro88Ser; see Figure 1a);

- alanine to serine at position 96 of connexin40 (Ala96Ser; see Figure 1d), or

- methionine to valine at position 163 of connexin40 (Met163Val; see figure 1b).

The nucleic acid array may comprise a full length connexin40 nucleic acid sequence encoding the one or more than one mutation, or it may comprise fragments of the connexin40 nucleic acid sequence encoding the one or more than one mutation. For example, the array may comprise separate nucleic acids sequences encoding mutations within one or more than one transmembrane domain, nucleic acid fragments that encode one or more than one mutation between amino acids 20-42, 76-98, 160-183 and 206-230 of connexin40, or nucleic acid fragments that encode glycine to aspartate at position 38 of connexin40 (Gly38Asp), proline to serine at position 88 of connexin40 (Pro88Ser), alanine to serine at position 96 of connexin40 (Ala96Ser), methionine to valine at position 163 of connexin40 (Met163Val), or a combination thereof. The nucleic acid array may comprise a nucleotides sequence or fragments of nucleotide sequences comprising a T at nucleotide position 262, an A at position 113, a G at position 487, a T at position 286, or a combination thereof, of connexin40 (SEQ ID NO:6, wt connexin40).

Therefore, according to the present invention there is provided a method of detecting cardiac arrhythmia, or the potential for cardiac arrhythmia, in a subject comprising, determining whether there is a mutation in the nucleotide sequence, the amino acid sequence, or both, of connexin40 obtained from the subject. The presence of the mutation may be determined using standard techniques as would be know to one of skill in the art (e.g. Sambrook et al., Molecular Cloning, A Laboratory Manual 2nd ed. 1989; Ausubel et al., eds., Current Protocols in Molecular Biology, 1994, which are incorporated herein by reference), for example using a method selected from the group consisting of:

- comparing the nucleotide sequence of connexin40 obtained from the subject to a nucleotide sequence of connexin40 obtained from a healthy patient using a sequence comparison, for example BLAST analysis using default parameters,

- comparing the nucleotide sequence of connexin40 obtained from the subject to a nucleotide sequence of connexin40 obtained from a healthy patient, or otherwise determining if there is a mutation in connexin40, using PCR,

- comparing the nucleotide sequence of connexin40 obtained from the subject to a nucleotide sequence of connexin40 obtained from a healthy patient, or otherwise determining if there is a mutation in connexin40, using LCR,

- comparing the nucleotide sequence of connexin40 obtained from the subject to a nucleotide sequence of connexin40 obtained from a healthy patient, or otherwise determining if there is a mutation in connexin40, using SNP analysis,

- comparing the nucleotide sequence of connexin40 obtained from the subject to a nucleotide sequence of connexin40 obtained from a healthy patient, or otherwise determining if there is a mutation in connexin40, using a nucleic acid array,

- comparing the amino acid sequence of connexin40 obtained from the subject to an amino acid sequence of connexin40 obtained from a healthy patient, or otherwise determining if there is a mutation in connexin40, using an epitope-based assay,

- comparing the amino acid sequence of connexin40 obtained from the subject to an amino acid sequence of connexin40 obtained from a healthy patient, or otherwise determining if there is a mutation in connexin40, using a monoclonal antibody,

- comparing the amino acid sequence of connexin40 obtained from the subject to an amino acid sequence of connexin40 obtained from a healthy patient, or otherwise determining if there is a mutation in connexin40, using an ELISA assay; and

- comparing the amino acid sequence of connexin40 obtained from the subject to an amino acid sequence of connexin40 obtained from a healthy patient, or otherwise determining if there is a mutation in connexin40, using western analysis.

With reference to Figures 3a and 4a, it can be seen that expression of nucleotide sequence encoding wild-type (wt) connexin40 (wtconnexin40; SEQ ID NO:6) in a gap junctional communication-deficient cell line, neuroblastoma (N2A, obtained from the ATCC) cells (see example 2), is localized at the cell membrane and localized to sites of cell-to-cell contact. Furthermore gap junction plaques are readily observed in N2A cells expressing wtconnexin40 (Figures 3a, 4a). These observations are noted with either the expression of wtconnexin40, or a wt connexin40 tagged with green fluorescence protein (wtconnexin40-GFP; Figure 3a, 4a).

However, expression of modified connexin40, comprising the Pro88Ser (Figures 3b, 4b), or Gly38Asp (Figures 4c, 4i) mutations, within N2A cells results in the distribution of connexin40 in a random manner throughout the cell with no gap junction formation. These results are indicative of impaired intracellular trafficking. Impaired intracellular trafficking is also observed when Pro88Ser connexin40 -GFP (a tagged mutant connexin40) is expressed in N2A cells (Figure 3c). Cells expressing Ala96Ser also demonstrated increased intracellular localization, however, gap junction plaques were present in similar amounts to wtconnexin40 (Figure 4e). These findings indicate that impaired trafficking of mutant connexin40 protein to the cell surface may provide a functional mechanism of deficient intercellular coupling causative for disease in patients harboring connexin40 mutations. Cells expressing Met163Val revealed both subcellular localization and gap junction plaque formation similar to wtconnexin40 (Figure 4g).

Co-expression of Pro88Ser connexin40-GFP (mutant connexin40) and untagged wtConnexin40, (wild type Connexin40) in N2A cells produced visible gap junction plaques at the cell membrane (Figure 3d). These results suggests that impaired intracellular trafficking associated with a mutant connexin40 comprising the Pro88Ser mutation is rescued in the presence of wtconnexin40. Without wishing to be bound by theory, these results suggest that these cells also produced heteromeric mutant-wt gap junctions.

Therefore, the present invention provides a method of detecting cardiac arrhythmia, or the potential for cardiac arrhythmia, in a subject comprising, determining whether intracellular trafficking is modified. If desired, a finding of modified intracellular trafficking may be followed by a determination as to whether there is a mutation in the nucleotide sequence of connexin40 (connexin40) as described above.

To assess the intercellular electrical coupling properties of wt and mutant connexin40 proteins, electrophysiological recordings were performed on paired N2A cells demonstrating visible gap junction plaques, when possible. No visible plaques were observed for cells expressing GFP-tagged Pro88Ser mutant connexin40 and consistently resulted in the absence of functional gap junction coupling (Figure 5a; Table 2, Example 3). This data associated with the Pro88Ser mutant is consistent with the cellular and electrophysiological findings of the parallel Cx50 disease-causing Pro88Ser mutation responsible for familial cataracts (Pal J.D., et. al., Am. J. Physiol. 276, C1443, 1999).

In cell pairs expressing Gly38Asp mutant protein, cell-cell coupling occurred at a significantly reduced junctional conductance (compared to wtconnexin40), despite the absence of visible gap junction plaques (Table 2, Example 3). In 2 cell pairs that demonstrated gap junction plaques, the coupling conductances were similar to those of wtconnexin40. Without wishing to be bound by theory, this latter result suggests that the functional consequence of theGly38Asp mutation may be predominantly related to impaired trafficking to the cell surface and reduced gap junction formation rather than impaired coupling properties.

Paired cells with visible gap junction plaques expressing Ala96Ser consistently demonstrated absent or weak cell-cell coupling compared to wtconnexin40 (Table 2, Example 3). Without wishing to be bound be theory, this finding suggests that the Ala96Ser mutation may significantly impair functional cell-cell coupling, in addition to demonstrating impaired trafficking to the cell surface.

Paired cells expressing Met163Val demonstrated cell coupling properties similar to wtConnexin40 (Table 2, Example 3). Without wishing to be bound by theory, this observation suggests that this mutation may represent a benign polymorphism.

The ability of wild-type human connexin40 and mutant connexin40 to induce gap junctional coupling in *Xenopus* oocyte pairs was investigated. In these experiments, *Xenopus* oocytes were injected with cRNA for wild-type connexin40, mutant connexin40, or a 1:1 mixture of wildtype and mutant. The Pro88Ser mutant completely inhibited the activity of co-expressed wild-type connexin40 (Figure 5c). Similarly, co-expression of Ala96Ser with wild-type connexin40 significantly reduced gap junctional conductance (Figure 5c).

The Ala96Ser mutation was the only mutation detected in lymphocytes as well as heart tissue, indicating this was a germline mutation. The connexin40 gene from the immediate family members of this affected individual was sequenced for this mutation. The mutation was absent from the patient's wife, but present in their 2 sons, ages 40 and 42, who did not yet have a history of atrial fibrillation. However, surface electrocardiography demonstrated significantly abnormal P-wave duration in the carrier sons, similar to their father's P wave prior to atrial fibrillation onset at age 41 (Figure 6). Surface electrocardiographic P-wave duration is a known predictor of atrial fibrillation (Dilaveris P.E., et. al., Am. Heart J. 135, 733; 1998). As is common with arrhythmias known to have a genetic cause, clinical latency for the condition is usual. Therefore, these results suggest that detection of a modification within the connexin40 sequence reveals a potential for developing cardiac arrhythmia.

Further functional data was obtained using electrophysiologic studies of paired cells co-expressing wtconnexin40 along with GFP, or expressing a fused wtconnexin40-GFP construct. As shown in Figure 5a, intercellular coupling and voltage-dependant gating is observed in cells expressing wtconnexin40 (along with, or fused to, GFP). Recordings from paired cells expressing mutant Pro88Ser connexin40 consistently resulted in the absence of functional gap junction coupling displaying impaired electrical coupling (Figure 5b; also see Example 3).

Therefore, the present invention provides a method of detecting cardiac arrhythmia, or the potential for cardiac arrhythmia, in a subject comprising, determining whether there is modified electrical coupling within a sample obtained from the subject. If desired, a finding of impaired electrical coupling may be verified by determining if there is a mutation in the nucleotide sequence of connexin40 (connexin40) as described above.

High-resolution electrical mapping studies during AF have documented multiple reentry circuits as a fundamental component for perpetuation of the arrhythmia (Konings K.T., et. al., Circulation 89, 1665, 1994;. Cox J.L., et. al., J. Thorac. Cardiovasc. Surg. 101, 406, 1991). The myocardial substrate for reentry initiation is dependant on regional variability in conduction velocity, creating a localized asymmetry in the depolarizing current wavefront through zones of tissue (Spach, M.S., & Josephson M.E., J. Cardiovasc. Electrophysiol. 5, 182, 1994). Such a substrate promotes reentry, whereby depolarizing wavefronts continually reenter themselves. Since cell-to-cell coupling of depolarizing current is mediated only at gap junctions, the biophysical properties of gap junctions are critical in ensuring normal myocardial conduction (Kanno, S., & Saffitz, J.E., Cardiovasc. Pathol. 10, 169, 2001).

Without wishing to be bound by theory, myocardium harboring a population of cells containing mutant connexin40 protein that adversely effects electrical coupling could give rise to heterogeneous regions of tissue conduction, providing an arrhythmogenic substrate predisposing the atria to micro-reentry arrhythmias. Studies of connexin40-deficient mice demonstrate intra-atrial conduction delay and an increased vulnerability to atrial arrhythmias, confirming connexin40 as an important determinant of normal atrial electrophysiology (Hagendorff, A., et. al., Circulation 99, 1508-1515, 1999; Verheule, Set. Al., J. Cardiovasc. Electrophysiol. 10, 1380-1389, 1999). Thus, modification of connexin40 function may prove more efficacious in the medical management of human atrial fibrillation than currently available therapy targeting sarcolemmal ion channels.

The uncommon occurrence of familial AF suggests that germline mutations of disease-causing genes may be associated with human embryonic lethality. In the context of connexin40 mutations, lethality might arise due to cardiac malformations, as have been observed in connexin40-deficient mice (Gu, H., et. al., Circ. Res. 93, 201-206, 2003). The data presented herein indicates that common diseases traditionally considered as "idiopathic", may have a genetic basis that is confined to the diseased tissue.

Cells, such as but not limited to neuroblastoma N2A cells, that have been modified to express Pro88Ser connexin40 or Pro88Ser connexin40-GFP (for example as described in Examples 2 and 3), may be used for the basis of a drug screening assay, to screen compounds that maybe useful in restoring gap junction plaque formation, intracellular trafficking, intracellular coupling, or a combination thereof. Any suitable method may be used to determine gap junction plaque formation, intracellular trafficking, or intracellular coupling, including visual and electrophysiolgical methods as described herein. However, it is to be understood that other methods known to one of skill in the art may also be used, including methods that may be used for screening a large volume of compounds using cell-based assays. Examples of such method include, but are not limited to that described in WO 03/063891 (which is incorporated herein by reference).

Therefore, the present invention also provides a method of identifying a compound for the treatment of cardiac arrhythmia comprising,

- providing a cell culture expressing a modified connexin40, the cell culture exhibiting impaired intracellular trafficking, impaired electrical coupling, reduced gap junction plaque formation, reduced intracellular coupling, or a combination thereof, when compared to a wild-type cell

- adding the compound to the cell culture; and

- determining if intracellular trafficking is improved, electrical coupling is improved, gap junction plaques are formed, intracellular coupling is improved, or a combination thereof, when compared to an untreated cell culture expressing the modified connexin40.

For example, the modified connexin40 may comprises one or more than one mutation within a transmembrane domain, one or more than one mutation between amino acids 20-42, 76-98, 160-183 and 206-230 of connexin40, or comprises one or more than one of the following substitutions: Pro88Ser, Met163Val, Ala96Ser, or Gly38Asp.

The isolation and characterization of a biologically active, modified connexin40, for example but not limited to connexin40 with one or more than one mutation within a transmembrane domain, one or more than one mutation between amino acids 20-42, 76-98, 160-183 and 206-230 of connexin40, or Pro88Ser connexin40, Met163Val connexin40, Ala96Ser connexin40 or Gly38Asp connexin40, provides a means for assaying for inhibitors and activators of gap junctions that comprise connexin40. Such activators and inhibitors identified using the above assay system can be used to further study intracellular trafficking, gap junction formation or intracellular coupling. Such activators and inhibitors are useful as pharmaceutical agents for treating diseases involving connexin40, including but not limited to cardiac arrhythmia. Modulators are also useful as protective agents to prevent arrhythmias. Furthermore, methods of detecting connexin40 modified nucleic acids and polypeptides are also useful for diagnostic applications for diseases involving abnormal gap junction activity as described above. Any compounds identified using the methods of the present invention may be formulated with appropriate pharmaceutically acceptable carriers and administered in any convenient manner, for example but not limited to, orally, by transdermal patch, by injection, or gene therapy, as would be known to one of skill in the art.

The present invention also provides nucleic acids and methods for treating cells that express modified connexin40, for example, cells expressing Pro88Ser connexin40, Met163Val connexin40, Ala96Ser connexin40 or Gly38Asp connexin40. Such methods may include the transfection of cells *in vitro* and *in vivo* with wild type connexin40, or a portion of wtconnexin40, for example that encodes Pro88, Met163, Ala96 or Gly38, as required. Nucleic acids encoding wtconnexin40, or a fragment thereof, can be inserted into any of a number of well-known vectors for the transfection of target cells and organisms as described below. The nucleic acids are transfected into cells, *ex vivo* or *in vivo,* through the interaction of the vector and the target cell. Expression of the wtconnexin40 or a portion thereof, under the control of a suitable promoter, thereby mitigates the effects of expression of the modified connexin40 gene. The compositions are administered to a patient in an amount sufficient to elicit a therapeutic response in the patient. For a review of gene therapy procedures see Anderson (Science 256:808-813, 1992), Nabel and Felgner (TIBTECH 11: 211-217, 1993), Mitani and Caskey (TIBTECH 11:162-166, 1993), Mulligan (Science 926-932, 1993), Dillon (TIBTECH 11:167-175, 1993), Miller (Nature 357:455-460, 1992), Van Brunt (Biotechnology 6(10):1149-1154, 1998), all of which are incorporated herein by reference.

Non-viral vector delivery systems include DNA plasmids, naked nucleic acid, and nucleic acid complexed with a delivery vehicle such as a liposome. Viral vector delivery systems include DNA and RNA viruses, which have either episomal or integrated genomes after delivery to the cell. Methods of non-viral delivery of nucleic acids include lipofection (e.g. US 5,049,386; US 4,946,787; U.S. 4,897,355, which are incorporated herein by refreence), microinjection, biolistics, virosomes, liposomes, immunoliposomes, polycation or lipid:nucleic acid conjugates, naked DNA, artificial virions, and agent-enhanced uptake of DNA as is known to one of skill in the art (see for example Kriegler, Gene Transfer and Expression: A Laboratory Manual (1990); and Current Protocols in Molecular Biology (Ausubel et al., eds., 1994; which are incorporated herein by reference).

Conventional viral based systems for the delivery of nucleic acids include retroviral, lentivirus, adenoviral, adeno-associated and herpes simplex virus vectors for gene transfer. Integration in the host genome is possible with the retrovirus, lentivirus, and adeno-associated virus gene transfer methods, often resulting in long-term expression of the inserted transgene. Additionally, high transduction efficiencies have been observed in many different cell types and target tissues. Widely used retroviral vectors include those based upon murine leukemia virus (MuLV), gibbon ape leukemia virus (GaLV), simian immunodeficiency virus (SIV), and combinations thereof (see, e.g., Buchscher et al., J. Virol. 66:2731-2739 (1992); Johann et al., J. Virol. 66:1635-1640 (1992); Sommerfelt et al., Virol. 176:58-59 (1990); Wilson et al., J. Virol. 63:2374-2378 (1989); Miller et al., J. Virol. 65:2220-2224 (1991)). pLASN and MFG-S are examples are retroviral vectors that have been used in clinical trials (Dunbar et al., Blood 85:3048-305 (1995); Kohn et al., Nat. Med. 1:1017-102 (1995); Malech et al., Proc. Natl. Acad. Sci. U.S.A. 94:22 12133-12138 (1997); which are incorporated herein by reference).

It may be desired that the vector be delivered with a high degree of specificity to a particular tissue type, for example cardiac atrial tissue. A viral vector may be modified to have specificity for a given cell type by expressing a ligand as a fusion protein with a viral coat protein on the viruses outer surface. The ligand is chosen to have affinity for a receptor known to be present on the cell type of interest.

Gene therapy vectors can be delivered in vivo by administration to an individual patient, typically by systemic administration (e.g., intravenous, intraperitoneal, intramuscular, subdermal, or intracranial infusion) or topical application. Alternatively, vectors can be delivered to cells *ex vivo,* such as cells explanted from an individual patient (e.g., lymphocytes, bone marrow aspirates, tissue biopsy) or universal donor hematopoietic stem cells, followed by reimplantation of the cells into a patient, usually after selection for cells which have incorporated the vector.

In mammalian host cells, a number of viral-based expression systems may be utilized. In cases where an adenovirus is used as an expression vector, the connexin40 gene product coding sequence of interest may be ligated to an adenovirus transcription-translation control complex, e.g., the late promoter and tripartite leader sequence. This chimeric gene may then be inserted in the adenovirus genome by *in vitro* or *in vivo* recombination. Insertion in a non-essential region of the viral genome (e.g., region E1 or E3) will result in a recombinant virus that is viable and capable of expressing the connexin40 gene product in infected hosts (e.g., See Logan and Shenk, 1984, Proc. Natl. Acad. Sci. USA 81, 3655-3659, which is incorporated herein by reference). Specific initiation signals may also be required for efficient translation of inserted connexin40 gene product coding sequences. These signals include the ATG initiation codon and adjacent sequences. In cases where an entire connexin40 gene, including its own initiation codon and adjacent sequences, is inserted into the appropriate expression vector, no additional translational control signals may be needed. However, in cases where only a portion of the connexin40 gene coding sequence is inserted, exogenous translational control signals, including, perhaps, the ATG initiation codon, must be provided. Furthermore, the initiation codon must be in phase with the reading frame of the desired coding sequence to ensure translation of the entire insert. These exogenous translational control signals and initiation codons can be of a variety of origins, both natural and synthetic. The efficiency of expression may be enhanced by the inclusion of appropriate transcription enhancer elements, transcription terminators, etc. (see Bittner, et al., 1987, Methods in Enzymol. 153, 516-544, which is incorporated herein by reference).

Therefore, the present invention also provides a method of restoring intracellular trafficking, or electrical coupling in cells that are deficient in gap junctions comprising, introducing a wild type connexin40 gene, or a portion thereof, into the cells, and expressing the wild type connexin40 gene, or a portion thereof within the cells.

The present invention will be further illustrated in the following examples.

## Examples

This invention relies on routine techniques in the field of recombinant genetics. Basic texts disclosing the general methods of use in this invention include Sambrook et al., Molecular Cloning, A Laboratory Manual (2nd ed. 1989); Kriegler, Gene Transfer and Expression: A Laboratory Manual (1990); and Current Protocols in Molecular Biology (Ausubel et al., eds., 1994).

*Study Subjects*

All samples used in this study were collected with informed consent and were approved for study by institutional review boards from the University of Western Ontario and the University of Ottawa Heart Institute.

The diagnosis of "idiopathic" atrial fibrillation was based upon the following criteria: i) the documentation of the arrhythmia on 12-lead electrocardiogram, ii) the absence of structural heart disease as determined by 2-dimensional echocardiographic imaging, and iii) no family history of cardiac arrhythmias.

## Example 1: Genomic DNA analysis

Genomic DNA was isolated from resected cardiac tissue from 15 "idiopathic" atrial fibrillation (AF) patients who had undergone surgery for disease management[10]. All patients developed AF at age < 55 years (range 33-54), and were refractory to multiple medications.

*Genomic DNA extraction*

Genomic DNA from cardiac tissue was extracted from formalin-fixed, paraffin-embedded tissue sections. Tissue was de-paraffinized in xylene, followed by a 98% ethanol wash. Samples were dried and re-suspended in 2X proteinase K digestion buffer with 250 $\mu$g/ml of proteinase K and incubated for 3 days at 55°C. DNA was then isolated using a phenol-chloroform extraction and re-suspended in sterile water. A second set of tissue sections had genomic DNA extracted using the DNAeasy Extraction Kit (Qiagen) for use in independent PCR reactions. Extraction of genomic DNA from peripheral blood lymphocytes was performed using the phenol-chloroform method in affected patients or the FlexiGene DNA kit (Qiagen) in normal controls.

All cloning experiments were performed using TOPO TA cloning system (Invitrogen Life Technologies) according to the manufacturer's recommendations. Plasmid clones were prepared using the Qiagen Miniprep Plasmid Purification system

Sequencing of the gene encoding connexin40 identified heterozygous mutations in cardiac tissue from 6 of 15 patients (Table 1).

**Table 1A: Connexin 40 mutation status and characteristics in patients with idiopathic atrial fibrillation** (Pt, patient. LA, left atrial free wall. LAA, left atrial appendage. LV, left ventricle. RA, right atrial free wall. RAA, right atrial appendage. RV, right ventricle. None, no mutations detected).

| Pt# | Sex | Age at onset | Source(s) of Samples | Nucleotide change | Amino Acid change |
|---|---|---|---|---|---|
| 1 | M | 47 | LA | None | none |
| 2 | M | 49 | RA | 882C T | (none) Asp294Asp |
| 3 | F | 48 | LAA, LA, RA | None | none |

(continued)

| Pt# | Sex | Age at onset | Source(s) of Samples | Nucleotide change | Amino Acid change |
|---|---|---|---|---|---|
| 4 | M | 36 | LA, RA | None | none |
| 5 | F | 48 | LA, RA, LV, WBC | 262C T | Pro88Ser |
| 6 | M | 41 | LA, RA | 286G T | Ala96Ser |
| 7 | M | 53 | LAA, LA, RA, WBC | 262C T | Pro88Ser |
| 8 | M | 46 | RA | None | none |
| 9 | F | 45 | RAA, RV | 213G A* 487A G* | Gly38Val Met163val |
| 10 | M | 38 | LAA, RA | None | none |
| 11 M | | 46 | LAA, RAA, RA, LV, WBC | 262C T | Pro88Ser |
| 12 M | | 50 | LA, RA | None | none |
| 13 | M | 33 | LAA | None | none |
| 14 M | | 43 | LAA, RAA, LV | 262C T 882C T | Pro88Ser (none) Asp294Asp |
| 15 | Male | 54 | LA | None | none |
| * Gly38Val and Met163Val were not detected from RV tissue sample. | | | | | |

*Mutation Screening*

Primers were designed to amplify the coding sequence of GJA5 (connexin40) which is present entirely within exon 2 of the gene. Initial mutation screening was performed by direct sequencing following nested PCR amplification of genomic DNA isolated from cardiac specimens. PCR amplicons were purified using the QiaQuick PCR Purification kit (Qiagen) and directly sequenced using BigDye Terminator on an ABI 377 or ABI 3100 sequencer (ABI). Detected mutations on sequencing chromatograms were confirmed by PCR amplification and sequencing of independently extracted genomic DNA. In addtion to GJA5, the coding regions for GJA1 (Cx43) and GJA7 (Cx45), which did not detect sequence variations, were also sequenced.

The somatic Pro88Ser mutation was further confirmed for patients 7 and 11 by 3 additional methods:

i) Since 2 mutations (P88S and A96S) were identified within the 2nd transmembrane region of connexin40, a mutation detection assay was developed for this region using denaturing gradient gel electrophoresis (DGGE). Using Win-Melt™ software (BioRad, Hercules, CA), the theoretical melting profile of a 254bp fragment spanning the TM2 region and the DNA sequence for P88 was determined. Only one low melt domain for this fragment in the presence of a 40 bpGCclamp attached to our reverse primer. Ten microliters of the primary PCR product was electrophoresed at 80V(constant) for 14hrs at 60 degrees C through a 10% acrylamide gel, with a 40-80% linear gradient of urea-formamide as a denaturant according to the manufacturer's directions using the DCode™ system (BioRad). Gels were silver stained and a characteristic banding pattern suggestive of mutation was noted for patients 7 and 11. Individual silver-stained bands were excised (using a fresh razor 2 blade for each band) and solubilized in 20ul ddH$_2$O at RT overnight. Five microliters of each extract was reamplified using the same primers (without GC-clamp) and sequenced;

ii) subcloning of the entire connexin40 coding sequence followed by restriction enzyme digestion with HypCH4 IV of positively selected clones liberated a novel 331 bp product caused by the 262C→T transition in the mutant allele, as detected on 2% agarose gel electrophoresis and confirmed by direct sequencing; and

iii) subcloning of a 254 bp PCR product with direct sequencing of multiple clones confirming the presence of mutant allele in a minority fraction of alleles (Table 1B).

To confirm that tissue specimens of patients 7 and 11 coincided with lymphocyte specimens, all respective samples were genotyped for the connexin40 flanking polymorphic markers DIS442, DIS305, DIS2707, and DIS2844. Consistent genotype profiles existed for all specimens specific to patient 7 or patient 11. Unique alleles at each marker were noted for each patient, confirming they are not descendents of a common founder. The somatic Gly38Asp and Met163Val mutations were also further confirmed by sublcloning of PCR products and direct sequencing to identity mutant clones (Table 1B).

**Table 1B: Connexin40 clones from patients with somatic mutation.**

| Pt# | Sex | Source of Samples | # of clones analyzed | Mutant Clones % |
|---|---|---|---|---|
| 7 | Male | LA | 24 | Pro88Ser: 5 (21%) |
| 9 | Female | RAA | 10 | Gly38Asp:2 (20%) Met163Val:3 (30%) |
| 11 | Male | RA | 38 | Pro88Ser:13 (34%) |
| Pt, patient. LA, left atrial free wall. RA, right atrial free wall. RAA, right atrial appendage. | | | | |

A common 262C→T mutation, predicting a Pro88Ser substitution, was identified in 4 patients (see Figure 1). Allelic subcloning from selected cardiac tissue specimens revealed a mutant allele frequency in the range of 14-22%. This represents a conservative estimate of the allele frequency from cardiac myocytes owing to the presence of various cell types in the tissue specimens (data not shown). An additional patient harbored 2 non-allelic mutations, as determined by subcloning, leading to Gly38Val (113G→A) and Met163Val (487A→G) substitutions. Sequencing of DNA from peripheral lymphocytes of these patients showed no evidence of mutation, indicating a somatic source of the genetic defects. Cardiac specimens from a sixth patient demonstrated a 286G→T mutation, predicting an Ala96Ser amino acid change. This mutation was also identified from lymphocyte DNA.

Cross-species comparison of connexin40 shows a > 80% homology of protein and protein-encoding DNA sequence, reflecting the evolutionary conservation of the connexin40 protein and gene structure (Figure 2).

**Example 2: Expression of modified connexin40 in neurobalstoma cells**

To determine the functional significance of connexin40 mutations, wild-type (wt) or mutant connexin40 proteins were expressed in a gap junctional communication-deficient cell line, neuroblastoma (N2A) cells, as described below.

*Expression of connexin40 constructs and expression in N2A cells*

Human wtconnexin40 coding sequence was amplified from commercially available genomic DNA (BD Biosciences). The PCR products were cloned into the TOPO 2.1 vector (Invitrogen). Using the wtconnexin40 clone as a template, Gly38Asp, Pro88Ser, Ala96Ser, and Met163Val mutations were introduced by site-directed mutagenesis using the QuickChange mutagenesis kit (Stratagene). Clones were sequenced completely to confirm the mutation and exclude any other sequence variants that may have been introduced during PCR amplification.

For expression studies, wt and connexin40 mutants were subcloned into the pcDNA3.1 (-) vector (Invitrogen). In order to engineer GFP-tagged wtconnexin40 and the connexin40 mutants, the pEGFP-N vector (Clontech) was used. DNA sequence analysis confirmed that the fusion constructs contained sequence encoding a 9 amino acid peptide linking wtconnexin40 or the connexin40 mutant sequences to GFP.

N2A cells (obtained from the ATCC) were grown in 35 mm culture dishes to 50-70% confluency in DMEM containing 10%FBS, 100 U/ml of penicillin and 100 $\mu$g/ml of streptomycin. Cells were transfected in Opti-MEM medium containing Lipofectamine 2000 and 2 $\mu$g of the connexin40 or mutant connexin40 expression vectors mixed with 0.5 $\mu$g of the pEGFP expression vector to denote cells that likely received the wild-type or mutant connexin. In other experiments, cells were transfected with 2$\mu$g of constructs encoding GFPtagged wtconnexin40 or the connexin40 mutant vectors. In all cases, after 48hs post-transfection cells were immunolabelled for microscopy, or studied using patch-clamp techniques.

*Immunofluorescent labeling and Confocal Microscopy*

N2A cells expressing wtconnexin40 or connexin40 mutants were fixed at -20◦C in 80% methanol/20% acetone for 20 min prior to immunolabeling with 1/100 dilution of rabbit anti-connexin40 antibody (Chemicon) followed by a 1/200 dilution of anti-rabbit IgG conjugated to Texas red (Jackson ImmunoResearch laboratories, Inc.). Immunolabeled N2A cells and cells expressing wtconnexin40 or connexin40 mutants tagged to GFP were imaged on a Zeiss LSM 510 META confocal microscope. Fluorescent and transmitted light images were acquired and stored under conditions where GFP was pseudo-colored green and Texas red was assigned a red color.

N2A cells expressing wtconnexin40 (SEQ ID NO:6; Figure 3a), or wtconnexin40 tagged with green fluorescent protein (wtconnexin40-GFP, insert) showed predominant subcellular localization of the fluorescent tagged Cconnexin40 to sites of cell-cell contact (Figure 3a, arrows) and is localized at the cell membrane. Furthermore gap junction plaques are readily observed in N2A cells expressing wtconnexin40 (Figures 3a, 4a). Gap junction plaques are found with either the expression of wtconnexin40, or a wt connexin40 tagged with green fluorescence protein (wtconnexin40-GFP; Figure 3a, 4a).

In contrast, N2A cells expressing mutant Pro88Ser connexin40 (Figures 3b, 4b), GFP-tagged mutant Pro88Ser connexin40 (Figure 3c), or Gly38Asp (Figures 4c, 4i) mutations, results in the distribution of connexin40 in a random manner throughout the cell with no gap junction formation. These results are indicative of impaired intracellular trafficking.

Cells expressing Ala96Ser also demonstrated increased intracellular localization, however, gap junction plaques were present in similar amounts to wtconnexin40 (Figure 4e). These findings indicate that impaired trafficking of mutant connexin40 protein to the cell surface may provide a functional mechanism of deficient intercellular coupling causative for disease in patients harboring connexin40 mutations. Cells expressing Met163Val revealed both subcellular localization and gap junction plaque formation similar to wtconnexin40 (Figure 4g).

Co-expression of tagged mutant connexin40 (mutant Pro88Ser connexin40-GFP) and untagged wtconnexin40, in N2A cells, mimicking the heterozygous cell condition, resulted in the formation of visible gap junction plaques at the cell membrane (Figure 3d). This observation suggests that impaired intracellular trafficking of the mutant connexin40 in the presence of wtconnexin40 is rescued, along with the formation of heteromeric mutant/wt gap junctions.

**Example 3: Electrophysiologic studies of paired cells**

Intercellular electrical coupling properties of wt, and mutant connexin40 proteins was assed using paired N2A cells demonstrating visible gap junction plaques as obtained using the methods described in Example 2.

*Electrophysiolgical recordings*

A coverslip with low density N2A cells was placed in a recording chamber and perfused with a solution containing (in mM): NaCl 140, KCl 5, CsCl 2, $CaCl_2$ 2, $MgCl_2$ 1, Hepes 5, D-glucose 5, pyruvate 2, and $BaCl_2$ 1, at pH=7.4. N2A cells were visualized via an inverted phase-contrast microscope (Leica DMIRB). GFP positive cell pairs were identified and selected for dual, whole cell, voltage-clamp recording (Axopatch 200B, Axon Intruments Inc.). To facilitate functional testing, cell pairs with visible plaques at the cell-cell junction were selected. Recordings were carried out at room temperature. The recording pipette had a resistence of 3-5 MΩ when filled with an internal solution containing (in mM): CsCl 130, EGTA 10, $CaCl_2$ 0.5, MgATP 3, $Na_2$ATP 2, and Hepes 10, at pH=7.2 with CsOH. The current signal was digitized at a sampling rate of 1-2 kHz via a Digidata 1322a (Axon Instruments Inc.) and analyzed using pClamp9 software. Each cell of a pair was initially held at a common holding potential of 0 mV. To evaluate junctional coupling, 300 msec hyperpolarizing pulses to -30 mV were applied to one cell, from the holding potential of 0 mV, to establish a transjunctional voltage gradient ($V_j$), while the junctional current was measured in the second cell. Macroscopic functional conductance ($g_j$) was calculated, as:

$$g_j = I_j/V_j,$$

where $I_j$ is the measured functional current and $V_j$ is the transjunctional voltage. Low coupled pairs of N2A cells, where unitary gap junction channel-mediated currents were readily identifiable, were analyzed further for all-point amplitude histograms and single channel conductances.

No visible plaques were observed for cells expressing GFP-tagged Pro88Ser mutant connexin40, resulting in the absence of functional gap junction coupling (Figure 5a; Table 2. Recordings from paired cells expressing mutant Pro88Sec Connexin40 consistently resulted in the absence of functional gap junction coupling displaying impaired electrical coupling (Figure 5b). Intercellular coupling and voltage-dependant gating is observed in cells expressing wtconnexin40 (along with, or fused to, GFP).

**Table 2. Dual whole-cell voltage clamp recordings from N2A cells transfected with human wtconnexin40 and Pro88Ser connexin40 mutant.**

| N2A cell transfection | | coupled/tested | $G_j \pm$ SEM (nS)* |
|---|---|---|---|
| Co-transfection with GFP | wt connexin40 | 16/31 | 17.5 $\pm$ 4.3 |
| | Pro88Ser connexin40 | 1 /30 | 1.0 |
| GFP-tagged | Wtconnexin40 | 29/29 | 16.5 $\pm$ 2.1 |
| | Pro88Ser | 0/18 | None |
| | Ala96Ser | 7/31 | 1.8 $\pm$ 0.5 |
| | Gly38Asp | 17/18 | 5.7 $\pm$ 1.6 |
| | Met163Val | 22/22 | 17.8 $\pm$ 2.6 |
| *$G_j$ represents the junctional conductance of coupled cell pairs only. | | | |

In cell pairs expressing Gly38Asp mutant protein, and the absence of visible gap junction plaques, cell-cell coupling occurred at a significantly reduced junctional conductance (compared to wtconnexin40; Table 2). In 2 cell pairs that demonstrated gap junction plaques, the coupling conductances were similar to those of wtconnexin40. Paired cells with visible gap junction plaques expressing Ala96Ser consistently demonstrated absent or weak cell-cell coupling compared to wtconnexin40 (Table 2). Paired cells expressing Met163Val exhibited cell coupling properties similar to wtconnexin40 (Table 2).

*Xenpous* oocytes

connexin40 mutants that exhibited electrophysiological evidence of impaired gap junctional coupling and wt connexin40were tested for their ability to induce gap junctional coupling in *Xenopus* oocyte pairs. In these experiments, *Xenopus* oocytes were injected with cRNA for wild-type connexin40, mutant connexin40, or a 1:1 mixture of wildtype and mutant as described below.

For *Xenopus* oocyte studies, sequence confirmed wt or mutant connexin40 PCR products were subcloned into the SP6TII RNA expression vector. The plasmids were linearized with *Sal* I, and capped RNAs were synthesized using the mMessage mMachine SP6 in vitro transcription kit (Ambion, Austin, TX) according to the manufacturer's instructions. The amount of RNA was quantitated by measuring the absorbance at 260 nm. Following injection of *Xenopus* oocytes with cRNA for wtconnexin40, mutant connexin40 or a 1:1 ratio of wt:mutant and incubation for 24-48 hours at 18° C, the oocytes were devitellinized and paired. Gap junctional coupling was measured using a double two-microelectrode-voltage-clamp technique 24 hours later.

Antisense-treated oocytes injected with Pro88Ser mutant completely inhibited the activity of co-expressed wild-type connexin40 (Figure 5c). Similarly, co-expression of Ala96Ser with wild-type connexin40 significantly reduced gap junctional conductance (Figure 5c).

The present invention has been described with regard to one or more embodiments. However, it will be apparent to persons skilled in the art that a number of variations and modifications can be made without departing from the scope of the invention as defined in the claims.

**Claims**

1. A method of detecting cardiac arrhythmia, or a potential for cardiac arrhythmia in a subject comprising, determining whether there is a mutation in the nucleotide sequence, the amino acid sequence, or both, of connexin40 obtained from the subject, wherein the mutation in the nucleotide sequence encodes a mutation and/or, the amino acid sequence, comprises a mutation wherein the mutation is selected from the group consisting of a Pro88Ser mutation, an Ala96Ser mutation, or a Gly38Asp mutation in connexin40 (SEQ ID NO: 1).

2. The method of claim 1, wherein the nucleotide sequence of connexin40 is determined from a blood sample, or a heart tissue sample, obtained from the patient.

3. A method of identifying a compound for the treatment of cardiac arrhythmia comprising,

- providing a cell culture expressing a modified connexin40, the modified connexin40 comprising a Pro88Ser mutation, a Ala96Ser mutation or a Gly38Asp mutation in SEQ ID NO: 1, the cell culture exhibiting impaired intracellular trafficking, impaired electrical coupling, reduced gap junction plaque formation, reduced intracellular coupling, or a combination thereof, when compared to a cell expressing wild-type connexin40 (SEQ ID NO: 1).
- adding the compound to the cell culture; and
- determining if intracellular trafficking is improved, electrical coupling is improved, gap junction plaques are formed, intracellular coupling is improved, or a combination thereof, when compared to an untreated cell culture expressing the modified Connexin40.

4. An isolated nucleic acid encoding a modified connexin40, the modified connexin40 comprising aPro88Ser mutation, an Ala98Ser mutation or a Gly38Asp mutation in SEQ ID NO: 1.

5. A nucleic acid array comprising one or more than one of the nucleic acid of claim 4.

6. A method of detecting cardiac arrhythmia, or a potential for cardiac arrhythmia a subject comprising, which is to be obtained from the subject the nucleic acid sample with the nucleic acid array of claim 5.

7. The method of claim 6, wherein the nucleic acid sample is obtained from blood or heart tissue of the subject.

8. Use of a nucleic acid encoding connexin40 (SEQ ID NO: 1) for the production of a medicament for restoring intracellular trafficking in a cell that is deficient in gap junctions, said cell comprising a Pro88Ser mutation, an Ala98Ser mutation or a Gly38Asp mutation in SEQ ID NO: 1.

9. The use of claim 8, wherein the nucleic acid encoding connexin40 is introduced in the cell using a vector.

10. A modified connexin40 protein produced from the isolated nucleic acid of claim 4.

11. An isolated, modified connexin40 protein comprising a Pro88Ser mutation, an Ala98Ser mutation or a Gly38Asp mutation in SEQ ID NO: 1.

**Patentansprüche**

1. Verfahren zum Erkennen von Herzrhythmusstörungen oder eines Potenzials für Herzrhythmusstörungen bei einem Subjekt, wobei das Verfahren das Ermitteln umfasst, ob eine Mutation in der Nukleotidsequenz, der Aminosäuresequenz oder beiden des Connexins 40, das von dem Subjekt erhalten wird, vorliegt, wobei die Mutation in der Nukleotidsequenz eine Mutation codiert und/oder die Aminosäuresequenz eine Mutation umfasst, wobei die Mutation aus der Gruppe ausgewählt ist, die aus einer Pro88Ser-Mutation, einer Ala96Ser-Mutation und einer Gly38Asp-Mutation im Connexin 40 (SEQ-ID Nr. 1) besteht.

2. Verfahren nach Anspruch 1, wobei die Nukleotidsequenz des Connexins 40 anhand einer vom Patienten erhaltenen Blutprobe oder Herzgewebeprobe ermittelt wird.

3. Verfahren zum Identifizieren einer Verbindung zur Behandlung von Herzrhythmusstörungen, wobei das Verfahren das Folgende umfasst:

- Bereitstellen einer Zellkultur, welche ein modifiziertes Connexin 40 exprimiert, wobei das modifizierte Connexin 40 eine Pro88Ser-Mutation, eine Ala96Ser-Mutation oder eine Gly38Asp-Mutation in SEQ-ID Nr. 1 umfasst, wobei die Zellkultur einen beeinträchtigten intrazellulären Transport, eine beeinträchtigte elektrische Kopplung, eine verringerte Bildung von Gap-Junction-Plaque, eine verringerte intrazelluläre Kopplung oder eine Kombination daraus aufweist, wenn sie mit einer Zelle verglichen wird, die wildes Connexin 40 (SEQ-ID Nr. 1) exprimiert;
- Hinzugeben der Verbindung zu der Zellkultur und
- Ermitteln, ob im Vergleich zu einer unbehandelten Zellkultur, welche das modifizierte Connexin 40 exprimiert, der intrazelluläre Transport verbessert wird, die elektrische Kopplung verbessert wird, Gap-Junction-Plaques gebildet werden, die intrazelluläre Kopplung verbessert wird oder eine Kombination daraus vorliegt.

4. Isolierte Nukleinsäure, welche ein modifiziertes Connexin 40 codiert, wobei das modifizierte Connexin 40 eine Pro88Ser-Mutation, eine Ala98Ser-Mutation oder eine Gly38Asp-Mutation in SEQ-ID Nr. 1 umfasst.

5. Nukleinsäure-Array, welches eine oder mehr als eine der Nukleinsäuren nach Anspruch 4 umfasst.

6. Verfahren zum Erkennen von Herzrhythmusstörungen oder eines Potenzials für Herzrhythmusstörungen bei einem Subjekt, wobei das Verfahren das Hybridisieren der Nukleinsäureprobe, die von dem Subjekt erhalten wird, mit dem Nukleinsäure-Array nach Anspruch 5 umfasst.

7. Verfahren nach Anspruch 6, wobei die Nuleinsäureprobe aus dem Blut oder Herzgewebe des Subjekts erhalten wird.

8. Verwendung einer Nukleinsäure, welche Connexin 40 (SEQ-ID Nr. 1) codiert, für die Herstellung eines Medikaments zur Wiederherstellung des intrazellulären Verkehrs in einer Zelle, welcher es an Gap-Junctions mangelt, wobei die Zelle eine Pro88Ser-Mutation, eine Ala98Ser-Mutation oder eine lky38Asp-Mutation in SEQ-ID Nr. 1 umfasst.

9. Verwendung nach Anspruch 8, wobei die Nukleinsäure, welche Connexin 40 codiert, unter Verwendung eines Vektors in die Zelle eingeführt wird.

10. Modifiziertes Connexin-40-Protein, welches aus der isolierten Nukleinsäure nach Anspruch 4 hergestellt ist.

11. Isoliertes modifiziertes Connexin-40-Protein, welches eine Pro88Ser-Mutation, eine Ala98Ser-Mutation oder eine Gly38Asp-Mutation in SEQ-ID Nr. 1 umfasst.


**Revendications**

1. Procédé de détection d'une arythmie cardiaque ou d'un potentiel d'arythmie cardiaque chez un sujet, comprenant la détermination de la présence éventuelle d'une mutation dans la séquence nucléotidique, la séquence d'acides aminés, ou les deux, de la connexine 40 provenant du sujet, dans lequel la mutation présente dans la séquence nucléotidique code pour une mutation et/ou la séquence d'acides aminés comprend une mutation, ladite mutation étant choisie dans le groupe constitué par une mutation Pro88Ser, une mutation Ala96Ser ou une mutation Gly38Asp dans la connexine 40 (SEQ ID N° 1).

2. Procédé selon la revendication 1, dans lequel la séquence nucléotidique de la connexine 40 est déterminée à partir d'un échantillon de sang ou d'un échantillon de tissu cardiaque prélevé chez le patient.

3. Procédé d'identification d'un composé destiné au traitement de l'arythmie cardiaque, comprenant les étapes consistant à :

  - préparer une culture cellulaire qui exprime une connexine 40 modifiée, ladite connexine 40 modifiée comprenant une mutation Pro88Ser, une mutation Ala96Ser ou une mutation Gly38Asp dans la SEQ ID N° 1, la culture cellulaire présentant un trafic intracellulaire altéré, un couplage électrique altéré, une formation de plaques jonctionnelles réduite, un couplage intracellulaire réduit, ou une combinaison de ceux-ci, par comparaison avec une cellule qui exprime la connexine 40 de type sauvage (SEQ ID N° 1) ;
  - ajouter le composé à la culture cellulaire ; et
  - déterminer si le trafic intracellulaire est amélioré, si le couplage électrique est amélioré, si des plaques jonctionnelles se forment, si le couplage intracellulaire est amélioré, ou une combinaison de ceux-ci, par comparaison avec une culture cellulaire non traitée exprimant la connexine 40 modifiée.

4. Acide nucléique isolé codant pour une connexine 40 modifiée, ladite connexine 40 modifiée comprenant une mutation Pro88Ser, une mutation Ala98Ser ou une mutation Gly38Asp dans la SEQ ID N° 1.

5. Réseau d'acides nucléiques comprenant un ou plusieurs acides nucléiques selon la revendication 4.

6. Procédé de détection d'une arythmie cardiaque ou d'un potentiel d'arythmie cardiaque chez un sujet, qui consiste à hybrider l'échantillon d'acide nucléique à prélever chez le sujet avec le réseau d'acides nucléiques selon la revendication 5.

7. Procédé selon la revendication 6, dans lequel l'échantillon d'acide nucléique provient du sang ou du tissu cardiaque du sujet.

8. Utilisation d'un acide nucléique codant pour la connexine 40 (SEQ ID N° 1) pour produire un médicament destiné à rétablir le trafic intracellulaire dans une cellule qui est déficiente en jonctions communicantes, ladite cellule comprenant une mutation Pro88Ser, une mutation Ala98Ser ou une mutation Gly38Asp dans la SEQ ID N° 1.

9. Utilisation selon la revendication 8, dans laquelle l'acide nucléique codant pour la connexine 40 est introduit dans la cellule en utilisant un vecteur.

10. Protéine connexine 40 modifiée produite à partir de l'acide nucléique isolé selon la revendication 4.

11. Protéine connexine 40 modifiée isolée comprenant une mutation Pro88Ser, une mutation Ala98Ser ou une mutation Gly38Asp dans la SEQ ID N° 1.

Figure 1

**a**

G38D

| | | | |
|---|---|---|---|
| **Human** | 1 | MGDWSFLGNFLEEVHKHSTVVGKVWLTVLFIFRMLVLGTAAESSWGDEQADFRCDTIQPG | 60 |
| **Wolf** | 1 | MGDWSFLGEFLEEVHKHSTVTGKVWLTVLFIFRMLVLGTAAESSWGDEQADFQCDTMQPG | 60 |
| **Rat** | 1 | MGDWSFLGEFLEEVHKHSTVTGKVWLTVLFIFRMLVLGTAAESSWGDEQADFRCDTIQPG | 60 |
| **Mouse** | 1 | MGDWSFLGEFLEEVHKHSTVTGKVWLTVLFIFRMLVLGTAAESSWGDEQADFRCDTIQPG | 60 |
| **Hamster** | 30 | ------------------------------FLXRMLVLGTAAESSWGDEQADFRCDTIQPG | 60 |

P88S    A96S

```
61  CQNVCYDQAFPISHIRYWVLQIIFVSTPSLVYMGHAMHTVRMQEKRKLREAERAKEVRGS  120
61  CQNVCYDQAFPISHIRYWVLQIIFVSTPSLVYMGHAMHTVRMQEKRNVREKERAKEA-GA  120
61  CQNVCYDQAFPISHIRYWVLQIIFVSTPSLVYMGHAMHTVRMQEKQKLREAERAKEVGGT  120
61  CQNVCYDQAFPISHIRYWVLQIIFVSTPSLVYMGHAMHTVRMQEKQKLRDAERAKEAHRT  120
61  CQNVCYDQAFPISHIRYWVLQIIFVSTPSLVYMGHAMHTVRMQEKQKLRDAERAKEAGAT  120
```

M163V

```
121  GSYEYPVAEKAELSCWEEGNGRIALQGTLLNTYVCSILIRTTMEVGFIVGQYFIYGIFLT  180
121  GSYEYPVAEKAELSCWEEVNGRIVLQGTLLLNTYVCSILIRTTMEVAFIVGQYLLYGIFLD  180
121  GTYEY-DAEKAELSCWKEVNGKIVLQGTLLNTYVCTLLIRTAMEVAFIVGQYLLYGIFLD  180
121  GAYEYPVAEKAELSCWKEVDGKIVLQGTLLNTYVCTLLIRTTMEVAFIVGQYLLYGIFLD  180
121  GSYEYPVAEKAELSCWKEVDGKIVLQGTLLNTYVCTLLIRTTMEVAFIVGQYLLYGIFLD  180
```

```
181  TLHVCRRSPCPHPVNCYVSRPTEKNVFIVFMLAVAALSLLLSLAELYHLGWKKIRQRFVK  240
181  TLHVCRRSPCPHPVNCYVSRPTEKNVFIVFMLAVAALSLFLSLAELYHLGWKKIRQRFVK  240
181  TLHVCRRSPCPHPVNCYVSRPTEKNVFIVFMMAVAGLSLFLSLAELYHLGWKKIRQRILAK  240
181  TLHVCRRSPCPHPVNCYVSRPTEKNVFIVFMLAVAGLSLFLSLAELYHLGWKKIRQRFGK  240
181  TLHVCRRSPCPHPVNCYVSRPTEKNVFIVFMLAVAALSLFLSLAELYHLGWKKIRQRFVK  240
```

```
241  PRQHMAKCQLSGPSVGIVQSCTPPPDFNQCLENGPGGKFFNPFSNNMASQQNTDNLVTEQ  300
241  SGQCMAECQLFGPSAGIVQNCTPPPDFNQCLKNGPGGKFFNPFSNRMASQQNTDNIATEQ  300
241  SRQG-DKHQLTGPSTSIVQGITPPPDFNQCLKNSPDEKFFSDFSNNMGSRRNPDPLATEP  300
241  SRQGVDKHQLFGPETSIVQSITPPPDFNQCLKNSSGEKFFSDFSNNMGSRRNPDALATGE  300
241  SRQGMDKQQILRPSBNIVQSITPPPDFNQCLRSRPGEKFTHDFSNNMGSRRNPDTTLATEP  300
```

```
301  VRGQEQTPGEGFIQVRYGQKPEVPNGVSPGHRLPHGYHSDKRRLSKASSKARSDDLSV  358
301  VQGQEPTPGEGFINTRYAQKPEVPNGASPGHRLPHGYQSDKRRLSKASSKARSDDLSV  358
301  VFNQEQTPEEGFIHTQYGQKPECPSGASAGHRLFPQGYHSDKRRLSKASSKARSDDLSV  358
301  VFNQEQTPGEGFIHMHYSQKPEVASGASAGHRLPQGYHSDKRRLSKASSKARSDDLSV  358
301  MFDQEFLTSRDGFIHMHYGQKPEFHNGASPGHRLPHGYHGDK----------------  341
```

**b**

| ggc → gac<br>Gly → Asp | ccc → tcc<br>Pro → Ser | gcc → tcc<br>Ala → Ser |
|---|---|---|
| 103 _____ 123 | 253 | 292 |
| ...........ctcgtgctgggcacagctgct........... | ...........gtctccacgccctctctggtgtacatgggccacgccatgc...... | |
| ...........ctggtgctgggcacagctgc........... | ...........gtgtccacaccatctttggtgtacatgggccacgcaatgc...... | |
| ...........ctggtcctcggcacagctgct........... | ...........gtgtccacaccatctctggtgtacatgggccacgccatgc...... | |
| ...........ctggtcctgggcacagctgct........... | ...........gtgtccacgccttctctagtgtacatgggccatgccatgc...... | |
| ...........ctggtcctgggcacagctgct........... | ...........gtgtccacaccgtctctggtctacatgggccatgccatgc...... | |

atg → gtg
Met → Val

```
478 _____ 498
...........cgcaccaccatggaggtgggc...........
...........cgcaccaccatggaggtgggc...........
...........cgcaccgtcatggaggtgggc...........
...........cgcaccaccatggaggtggcc...........
...........cgcaccaccatggagtggct...........
```

# Figure 2

21

CACNAGCCATTTGGCGATGGAGCTTCTGGGAAATTCCTGGAGGAAGTACACAAGCACTC

GACCGTGGTAGGCAGGGTCTGGCTCACTGTCCTCTTCATATTCCGTATGCTCGTGCTGG

GCACAGCTGCTGAGTCTTCCTGGGGGGATGAGCAGGCTGATTTCCGGTGTGATACGATT

CAGCCTGGCTGCCAGAATGTCTGCTACGACCAGGCTTTCCCCATCTCCCACATTCGCTA

CTGGGTGCTGCAGATCATCTTCGTCTCCACGCCCTCTCTGGTGTACATGGGCCACGCCA

TGCACACTGTGCGCATGCAGGAGAAGCGCAAGCTACGGGAGGCCGAGAGGGCCAAAGAG

GTCCGGGGCTCTGGCTCTTACGAGTACCCGGTGGCAGAGAAGGCAGAACTGTCCTGCTG

GGAGGAAGGGAATGGAAGGATTGCCCTCCAGGGCACTCTGCTCAACACCTATGTGTGCA

GCATCCTGATCCGCACCACCATGGAGGTGGGCTTCATTGTGGGCCAGTACTTCATCTAC

GGAATCTTCCTGACCACCCTGCATGTCTGCCGCAGGAGTCCCTGTCCCCACCCGGACAA

CTGTAACGTATCCCGGTCCCACAGAGAAGAACGTCTNCACTGNCTTNATGCTGGCTGGG

GNNGCACTGACCCNCCNCCNTAGNCNGGCTGAACNCTACCACCTGGGCTGGAAGAAGAT

CAGACAGCGATTNGGCAAACCGNGGNAGCACANGGCTAAGTGCCAGCNANCNGGNCCCT

CTGNGGGCATAGNCCAGAGCNGNACACCANCCCCCGACNAAAAAACAGNGCCNGGAGAA

NGGNCCNGGGGGGAAAAAACNACAAACCCTNAAGNNAAAAAAAAGGCCNCCCAACAAAAC

ACAGACAACCNGGNCNCCGANCAAGNACGAGGGCAGGAGCAGAACNCCGGGGGAAGGNA

ACNNCCNGGANCGAAAAGGNCAGAANCNNGANGGNGCCAAAGGAN!

**Figure 2 c**

NACNAGCCANTTGGCGATGGAGCTNCTGGGAAATTCCTGGAGGAAGTACACAAGCACTC

GACCGTGGTAGGCAAGGCTCTGGCTCACTGTCCTCTTCATATTCCGTATGCTCGTGCTG

GGCACAGCTGCTGAGTCTTCCTGGGGGGATGAGCAGGCTGATTTCCGGTGTGATACGAT

TCAGCCTGGCTGCCAGAATGTCTGCTACGACCAGGCTTTCCCCATCTCCCACATTCGCT

ACTGGGTGCTGCAGATCATCTTCGTCTCCACGTCCTCTCTGGTGTACATGGGCCACGCC

ATGCACACTGTGCGCATGCAGGAGAAGCGCAAGCTACGGGAGGCCGAGAGGGCCAAAGA

GGTCCGGGGCTCTGGCTCTTACGAGTACCCGGTGGCAGAGAAGGCAGAACTGTCCTGCT

GGGAGGAAGGGAATGGAAGGATTGCCCTCCAGGGTCACTCTGCTCAACACCTATGTGTG

CAGCATCCTGATCCGCACCACCATGGAGGTGGGCTTCATTGTGGGCCAGTACTTCATCT

ACGGAATCTTCCTGACCACCCTGCATGTCTGCCGCAGGAGTCCCTGGCCCCACCGGACA

ACTGTAACGTATCCCGGCCCACAGAGAAGAATGTCTCCATGTCTTAAAGCTGGCTGNGG

NNGCACTGCCCCNCCTCCTNAGCCNGNCTGAACNCNACCANCTGGGCTGGAAGAAGATC

AGACAGCGATTGGACAAANCGNGGCAGCACANGGNNAAGTGCCAGTCNNNCAGGCCCCN

CAGGGGGCATAGACCAGAGCAGNACGCCANCCCCCGANAAAAANAGGGNCAGNAGAAAG

GCCCNGGGGGAAAAAACCANATNCCCNNCAGTAANAAAGANGGGNCNCCCAACAAAANA

CAGAAAAANCNGGNCACCGAATCAAGGACGAGGACAGGAGCNGACACCGGGGGAAGGAA

TNANCCAGGANCGTNAAGGTCAGAATCCNGAGGAGCCAAAGGAGN

**Figure 2d**

# Figure 3

**Figure 4**

# Figure 5

**Figure 6**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 0219966 A, Donahue J.K. and Marban E.
- WO 03063891 A
- US 5049386 A
- US 4946787 A
- US 4897355 A

### Non-patent literature cited in the description

- **Chen, Y.H.** *Science,* 2003, vol. 299, 251-254
- **Brugada, R.** *N. Engl. J. Med.,* 1997, vol. 336, 905-911
- **Ellinor, P.T.** *Circulation,* 2003, vol. 107, 2880-2883
- **Kanno, S. ; Saffitz, J.E.** *Cardiovasc. Pathol.,* 2001, vol. 10, 169-177
- **Huub, M.W.** *Cardiovascular Research,* 2002, vol. 54, 270-279
- **Alcolea, S.** *Circ Res.,* 2004, vol. 94, 100-109
- **Ri et al.** *Biophysical Journal,* 1999, vol. 76, 2887-2898
- **Suchyna et al.** *Nature,* 1993, vol. 365, 847-849
- **Suchyna, T.M.** *Nature,* 1993, vol. 365, 847
- **Nelis E.** *Hum. Mutat.,* 1997, vol. 9, 47
- **Janssen E.A.** *Hum. Genet.,* 1997, vol. 99, 501
- **Bort, S.** *Hum. Genet.,* 1997, vol. 99, 746
- **Shiels A.** *Am. J. Hum. Genet.,* 1998, vol. 62, 526
- **Kuntzer T.** *J. Neurol. Sci.,* 2003, vol. 207, 77
- **Marlin, S.** *Arch. Otolaryngol. Head Neck Surg.,* 2001, vol. 127, 927
- **Bayazit, Y.A.** *Int. J. Pediatr. Otorhinolaryngol.,* 2003, vol. 67, 1331
- **Krutovskikh, V. ; Yamasaki, H.** *Mutat. Res.,* 2000, vol. 462, 197
- **Feinberg, W.M.** *Arch. Intern Med.,* 1995, vol. 155, 469-473
- **Kelley, P.M.** *Am. J. Hum. Genet.,* 1998, vol. 62, 792-799
- **Bone, L.J.** *Neurology,* 1995, vol. 45, 1863-1866
- **Kanno, S. ; Saffitz, J.E.** *Cardiovasc. Pathol.,* 2001, vol. 10, 169
- **Sambrook et al.** Molecular Cloning, A Laboratory Manual. 1989
- Current Protocols in Molecular Biology. 1994
- **Pal J.D.** *Am. J. Physiol.,* 1999, vol. 276, C1443
- **Dilaveris P.E.** *Am. Heart J.,* 1998, vol. 135, 733
- **Konings K.T.** *Circulation,* 1994, vol. 89, 1665
- **Cox J.L.** *J. Thorac. Cardiovasc. Surg.,* 1991, vol. 101, 406
- **Spach, M.S. ; Josephson M.E.** *J. Cardiovasc. Electrophysiol.,* 1994, vol. 5, 182
- **Hagendorff, A.** *Circulation,* 1999, vol. 99, 1508-1515
- **Verheule, S.** *J. Cardiovasc. Electrophysiol.,* 1999, vol. 10, 1380-1389
- **Gu, H.** *Circ. Res.,* 2003, vol. 93, 201-206
- **Anderson.** *Science,* 1992, vol. 256, 808-813
- **Nabel ; Felgner.** *TIBTECH,* 1993, vol. 11, 211-217
- **Mitani ; Caskey.** *TIBTECH,* 1993, vol. 11, 162-166
- **Mulligan.** *Science,* 1993, 926-932
- **Dillon.** *TIBTECH,* 1993, vol. 11, 167-175
- **Miller.** *Nature,* 1992, vol. 357, 455-460
- **Van Brunt.** *Biotechnology,* 1998, vol. 6 (10), 1149-1154
- **Kriegler.** Gene Transfer and Expression: A Laboratory Manual. 1990
- **Buchscher et al.** *J. Virol.,* 1992, vol. 66, 2731-2739
- **Johann et al.** *J. Virol.,* 1992, vol. 66, 1635-1640
- **Sommerfelt et al.** *Virol.,* 1990, vol. 176, 58-59
- **Wilson et al.** *J. Virol.,* 1989, vol. 63, 2374-2378
- **Miller et al.** *J. Virol.,* 1991, vol. 65, 2220-2224
- **Dunbar et al.** *Blood,* 1995, vol. 85, 3048-305
- **Kohn et al.** *Nat. Med.,* 1995, vol. 1, 1017-102
- **Malech et al.** *Proc. Natl. Acad. Sci. U.S.A.,* 1997, vol. 94 (22), 12133-12138
- **Logan ; Shenk.** *Proc. Natl. Acad. Sci. USA,* 1984, vol. 81, 3655-3659
- **Bittner et al.** *Methods in Enzymol.,* 1987, vol. 153, 516-544